# EUROPEAN PATENT APPLICATION

(11) **EP 4 424 382 A1**
(43) Date of publication of application: **04.09.2024**
(21) Application number: 22886931.9
(22) Date of filing: 24.10.2022
(51) Int. Cl.: A61Q 5/00, A61Q 5/12, A61K 8/06, A61K 8/81, A61K 8/898

(54) **HAIR COSMETIC COMPOSITION**

(30) Priority: 25.10.2021 JP 2021174048; 27.07.2022 JP 2022119767
(71) Applicant: Kao Corporation, Tokyo 103-8210 (JP)
(72) Inventor: MIYOSHI, Eisuke, Tokyo 131-8501 (JP); UENO, Shiori, Tokyo 131-8501 (JP); SARUKAWA, Yuri, Tokyo 131-8501 (JP); KAWAMOTO, Koichi, Tokyo 131-8501 (JP)
(74) Representative: Hoffmann Eitle
(86) International application number: PCT/JP2022/039465
(87) International publication number: WO 2023/074604

(57) **Abstract**

Provided is a hair cosmetic composition containing: component (A): one or more polymers selected from the group consisting of a cationic polymer and an amphoteric polymer; component (B): one or more anionic polymers selected from the group consisting of a polyacrylic acid, a (meth)acrylic acid/alkyl (meth)acrylate copolymer, and a salt thereof; and component (C): one or more modified silicones selected from the group consisting of an amino-modified silicone and an aminopolyether-modified silicone, in which the component (B) has a weight average molecular weight of 3,000 or more and 50,000 or less, a total content of the component (A) and the component (B) is less than 1.0% by mass, and a content of the component (C) is 0.03% by mass or more.

## Description

### Field of the Invention

The present invention relates to a hair cosmetic composition.

### Background of the Invention

Hair cosmetic compositions containing a cationic polymer and an anionic polymer are known.

For example, JP 53-139734 A (PTL 1) describes that by using a composition for treating a keratin material, which is characterized by containing at least one anionic polymer and at least one cationic polymer in a solvent medium, the anionic polymer can be fixed to a keratin material that can particularly include hair, skin, or nails. In addition, it is assumed that a complex formed by an interaction between the anionic polymer and the cationic polymer is related to the reason why the above-described effect is obtained.

JP 2005-166333 A (PTL 2) describes that a hair cosmetic containing a cationic polymer having a charge density in a predetermined range and an anionic polymer containing 80% by mass or more and 100% by mass or less of a predetermined anionic constituent unit and having a weight average molecular weight of 4,000 or more and 80,000 or less can improve the feel and appearance of damaged hair, and these effects can be maintained even by repeated washing of the hair.

### Summary of the Invention

The present invention relates to a hair cosmetic composition containing:
component (A): one or more polymers selected from the group consisting of a cationic polymer and an amphoteric polymer;
component (B): one or more anionic polymers selected from the group consisting of a polyacrylic acid, a (meth)acrylic acid/alkyl (meth)acrylate copolymer, and a salt thereof; and
component (C): one or more modified silicones selected from the group consisting of an amino-modified silicone and an aminopolyether-modified silicone,
in which the component (B) has a weight average molecular weight of 3,000 or more and 50,000 or less, a total content of the component (A) and the component (B) is less than 1.0% by mass, and a content of the component (C) is 0.03% by mass or more.

### Detailed Description of the Invention

As described in PTL 1, it is known to use a complex formed from a cationic polymer and an anionic polymer (hereinafter, also referred to as "polyion complex") as a composition for treating a keratin substance, but there is still room for improvement in terms of performance improvement when used as a hair cosmetic composition.

For example, in a hair conditioner which is a type of hair cosmetic composition, it is required that the feel when applied to the hair is good, and that the hair after treatment does not spread even under high humidity conditions and has excellent moisture resistance. In particular, regarding the suppression of spread of the hair under high humidity conditions, no particular effect has been obtained in the prior art according to PTLs 1 and 2.

In addition, as a composition containing a polyion complex, there is a composition in the form of an oil-in-water type or water-in-oil type emulsion composition, but in these emulsion composition, improvement in stability with time has been a problem. In particular, in a hair cosmetic composition, a cationic surfactant, a higher alcohol, or the like may be blended in order to improve the feel of hair after treatment. However, there has been a problem that when these components are blended in an emulsion composition containing a polyion complex, the stability is likely to be lowered.

The present invention addresses the problem of providing a hair cosmetic composition which has a good feel at the time of application to the hair, can suppress the spread of the hair under high humidity conditions, and has high stability.

The present inventors have found that a hair cosmetic composition containing one or more polymers selected from the group consisting of a cationic polymer and an ampholytic polymer, a predetermined anionic polymer, and a predetermined modified silicone, in which the total content of the two polymers is less than a predetermined value, can solve the above problem.

According to the present invention, a hair cosmetic composition which has a good feel at the time of application to the hair, can suppress the spread of the hair under high humidity conditions, and has high stability can be provided.

### [Hair Cosmetic Composition]

The hair cosmetic composition of the present invention (hereinafter, also simply referred to as "the composition of the present invention") contains:
component (A): one or more polymers selected from the group consisting of a cationic polymer and an amphoteric polymer;
component (B): one or more anionic polymers selected from the group consisting of a polyacrylic acid, a (meth)acrylic acid/alkyl (meth)acrylate copolymer, and a salt thereof; and
component (C): one or more modified silicones selected from the group consisting of an amino-modified silicone and an aminopolyether-modified silicone,
in which the component (B) has a weight average molecular weight of 3,000 or more and 50,000 or less, a total content of the component (A) and the component (B) is less than 1.0% by mass, and a content of the component (C) is 0.03% by mass or more.

By having the above constitution, the hair cosmetic composition of the present invention has a good feel at the time of application to the hair, can suppress the spread of the hair under high humidity conditions, and has high stability.

In the description herein, "containing the component X" also includes the blending of the component X.

Although the reason why the hair cosmetic composition of the present invention exhibits the above effect is not clear, it is considered as follows.

The hair cosmetic composition of the present invention contains a polyion complex formed from the component (A) and the component (B).

The polyion complex is an aggregate formed by a chemical electrostatic interaction between the component (A) and the component (B). The polyion complex is poorly watersoluble, and a hydrophobic film can be formed by applying a hair cosmetic composition containing the polyion complex to hair. It is considered that the formation of this hydrophobic film improves the feel at the time of application to the hair and also improves the moisture resistance. Furthermore, it is considered that the component (C) contained in the hair cosmetic composition coats the hair surface, thereby further improving the effect of suppressing the spread of the hair under high humidity conditions.

In addition, by setting the total content of the component (A) and the component (B) in the hair cosmetic composition to be less than 1.0% by mass, it is considered that the hair cosmetic composition becomes stable even in the form of an emulsion composition containing a polyion complex.

The form of the hair cosmetic composition of the present invention is preferably an emulsion composition, and more preferably an oil-in-water type emulsion composition from the viewpoint of stabilization of the composition and from the viewpoint of improvement in feeling in use.

Examples of the product form of the hair cosmetic composition include a hair shampoo, a hair rinse, a hair conditioner, a hair treatment (including a type that is not washed away), and a hair styling agent. Among these, from the viewpoint of the effectiveness of the effect of the present invention, a hair conditioner, a hair treatment, or a hair styling agent is preferable.

Hereinafter, each component contained in the hair cosmetic composition will be described.

### (Component (A): One or more polymers selected from the group consisting of cationic polymers and amphoteric polymers)

The component (A) is one or more polymers selected from the group consisting of cationic polymers and amphoteric polymers, which can form a polyion complex by interaction with the component (B).

The cationic polymer as the component (A) preferably means a polymer having a cationic group and substantially having no anionic group and amphoteric group. The phrase "substantially having no anionic group and amphoteric group" means that the molar amount of the anionic group and the amphoteric group with respect to the cationic group is preferably 0.1% or less.

The amphoteric polymer as the component (A) is preferably a polymer having a cationic group and an anionic group, and the pH of a 1% aqueous solution of the polymer at 25°C is less than 5.1, and the polymer is positively charged as the total charge of the polymer. The pH can be measured with a pH meter.

The cationic group in the description herein is a cationic group or a group that can be ionized to become a cationic group, and specific examples thereof include a primary amino group, a secondary amino group, a tertiary amino group, and a quaternary ammonium group.

The anionic group is an anion group or a group that can be ionized to form an anion group, and specific examples thereof include one or more selected from the group consisting of acidic groups such as a carboxy group, a sulfonic acid group, and a phosphoric acid group, preferably one or more selected from a carboxy group and a sulfonic acid group, and more preferably a carboxy group. At least a part of the anionic groups may be neutralized and in the form of a salt.

The cationic charge density of the component (A) is preferably 0.1 meq/g or more, more preferably 0.5 meq/g or more, still more preferably 1.0 meq/g or more, and even more preferably 2.0 meq/g or more, from the viewpoint of facilitating the formation of the polyion complex by interaction with the component (B), and is preferably 10 meq/g or less, more preferably 8.0 meq/g or less, and still more preferably 7.0 meq/g or less, from the viewpoint of stability of the composition. Thus, the cationic charge density of the component (A) is preferably 0.1 meq/g or more and 10 meq/g or less, more preferably 0.5 meq/g or more and 8.0 meq/g or less, still more preferably 1.0 meq/g or more and 7.0 meq/g or less, and even more preferably 2.0 meq/g or more and 7.0 meq/g or less.

The cationic charge density of the component (A) can be calculated from (the number of moles of cationic groups contained per 1 g of polymer) × 1000 (meq/g).

The hair cosmetic composition may use two or more polymers as the component (A), and in this case, the cationic charge density of the component (A) is obtained by calculating the weighted average from the cationic charge density and the blending amount of each polymer.

The weight average molecular weight (Mw) of the component (A) is preferably 5,000 or more, and more preferably 8,000 or more, and is preferably 2,000,000 or less, more preferably 1,500,000 or less from the viewpoints of facilitating the formation of the polyion complex, stability of the composition, improvement in feel at the time of application to the hair, and suppression of spread of the hair under high humidity conditions. Thus, the weight average molecular weight (Mw) of the component (A) is preferably 5,000 or more and 2,000,000 or less, and more preferably 8,000 or more and 1,500,000 or less.

The weight average molecular weight of the component (A) can be measured by gel permeation chromatography (GPC).

Specific examples of the polymer used as the component (A) include a cationized guar gum, a cationized tara gum, a cationized locust bean gum, a cationic starch, a cationized cellulose, a cationized hydroxyalkyl cellulose, a cationized polyvinyl alcohol, a polyethyleneimine, a quaternized dialkylaminoalkyl (meth)acrylate polymer, a diallyl quaternized ammonium salt polymer, a methacrylamidopropyltrimethylammonium salt polymer, a methacryloyl ethyltrimethylammonium salt polymer, a vinyl imidazolium trichloride-vinylpyrrolidone copolymer (polyquaternium-16), a vinyl pyrrolidone-alkylamino(meth)acrylate copolymer, a vinyl pyrrolidone-alkylamino(meth)acrylate-vinyl caprolactam copolymer, an alkyl acrylamide-(meth)acrylate-alkylamino alkylacrylamide-polyethylene glycol (meth)acrylate copolymer, and an adipic acid-dimethylaminohydroxypropyl ethylenetriamine copolymer. One, or two or more of these can be used.

Among the above, examples of the cationized hydroxyalkyl cellulose include cationized hydroxyethyl cellulose and cationized hydroxypropyl cellulose, and examples of the cationized hydroxyethyl cellulose include o-[2-hydroxy-3-(trimethylammonio)propyl]hydroxyethyl cellulose chloride (polyquaternium-10).

Examples of the quaternized dialkylaminoalkyl (meth)acrylate polymer include a vinylpyrrolidone-N,N-dimethylaminoethyl methacrylate diethyl sulfate copolymer (polyquaternium-11), and an N,N-dimethylaminoethyl methacrylate diethyl sulphate-N,N-dimethylacrylamide-polyethylene glycol dimethacrylate copolymer (polyquaternium-52).

Examples of the diallyl quaternized ammonium salt polymer include a dimethyldiallylammonium chloride polymer (polyquaternium-6), a dimethyldiallylammonium chloride-acrylic acid copolymer (polyquaternium-22), a diallyldimethylammonium chloride-acrylamide copolymer (polyquaternium-7), and an acrylamide-acrylic acid-dimethyldiallylammonium chloride copolymer (polyquaternium-39).

Examples of the methacrylamidopropyltrimethyl ammonium salt polymer include a methacrylamidopropyltrimethyl ammonium chloride polymer, a vinylpyrrolidone-methacrylamidopropyltrimethyl ammonium chloride copolymer, an acrylic acid-methyl acrylate-methacrylamidopropyltrimethyl ammonium chloride copolymer (polyquaternium-47), and an acrylic acid-acrylamide-methacrylamidopropyltrimethyl ammonium chloride copolymer (polyquaternium-53).

Examples of the methacryloyl ethyl trimethyl ammonium salt polymer include a methacryloyl ethyl trimethyl ammonium chloride polymer (polyquaternium-37), a methacryloyl ethyl dimethyl betaine-methacryloyl ethyl trimethyl ammonium chloride-methoxypolyethylene glycol methacrylate copolymer (polyquaternium-49), and a methacryloyl ethyl dimethyl betaine-methacryloyl ethyl trimethyl ammonium chloride-2-hydroxyethyl methacrylate copolymer (polyquaternium-48).

The component (A) can be used alone or in combination of two or more thereof. Among the above, from the viewpoints of facilitating the formation of the polyion complex, stability of the composition, improvement in feel at the time of application to the hair, and suppression of spread of the hair under high humidity conditions, the component (A) is at least a polymer having a cationic group.

From the viewpoints of stability of the composition, improvement in feel at the time of application to the hair, and suppression of spread of the hair under high humidity conditions, the component (A) is more preferably at least one selected from the group consisting of a quaternized dialkylaminoalkyl (meth)acrylate polymer, a diallyl quaternized ammonium salt polymer, a methacrylamidopropyl trimethylammonium salt polymer, a methacryloyl ethyl trimethylammonium salt polymer, and a vinyl imidazolium trichloride-vinyl pyrrolidone copolymer (polyquaternium-16), still more preferably one or more selected from the group consisting of a diallyl quaternized ammonium salt polymer, a methacrylamidopropyl trimethylammonium salt polymer, and a vinyl imidazolium trichloride-vinyl pyrrolidone copolymer (polyquaternium-16), even more preferably one or more selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), an acrylic acid-methyl acrylate-methacrylamidopropyl trimethylammonium chloride copolymer (polyquaternium-47), and a vinyl imidazolium trichloride-vinyl pyrrolidone copolymer (polyquaternium-16), and even more preferably one or more selected from the group consisting of a dimethyldiallylammonium chloride polymer (polyquaternium-6), an acrylamide-acrylic acid-dimethyldiallylammonium chloride copolymer (polyquaternium-39), and a vinyl imidazolium trichloride-vinyl pyrrolidone copolymer (polyquaternium-16).

Among the above, the preferable ranges of the cationic charge density and the weight average molecular weight of the following polymers are preferably as follows from the viewpoints of facilitating the formation of the polyion complex, stability of the composition, improvement in feel at the time of application to the hair, and suppression of spread of the hair under high humidity conditions.

The cationic charge density of the dimethyldiallylammonium chloride polymer (polyquaternium-6) is even more preferably 2.0 meq/g or more, and even more preferably 2.5 meq/g or more, and is even more preferably 6.5 meq/g or less. The weight average molecular weight is still more preferably 10,000 or more and 200,000 or less.

The cationic charge density of the dimethyldiallylammonium chloride-acrylic acid copolymer (polyquaternium-22) is even more preferably 2.0 meq/g or more, even more preferably 3.0 meq/g or more, and even more preferably 4.0 meq/g or more, and is even more preferably 6.5 meq/g or less, and even more preferably 6.0 meq/g or less. The weight average molecular weight is still more preferably 20,000 or more, even more preferably 50,000 or more, even more preferably 100,000 or more, even more preferably 200,000 or more, and even more preferably 300,000 or more, and is still more preferably 1,000,000 or less, even more preferably 600,000 or less, and even more preferably 500,000 or less.

The cationic charge density of the acrylamide-acrylic acid-dimethyldiallylammonium chloride copolymer (polyquaternium-39) is even more preferably 2.0 meq/g or more, and even more preferably 2.5 meq/g or more, and is even more preferably 6.5 meq/g or less, even more preferably 5.0 meq/g or less, and even more preferably 4.0 meq/g or less. The weight average molecular weight is still more preferably 20,000 or more, even more preferably 50,000 or more, and even more preferably 100,000 or more, and is still more preferably 1,000,000 or less, even more preferably 600,000 or less, even more preferably 500,000 or less, and even more preferably 300,000 or less.

The cationic charge density of the acrylic acid-methyl acrylate-methacrylamidopropyltrimethylammonium chloride copolymer (polyquaternium-47) is even more preferably 2.0 meq/g or more, and even more preferably 2.5 meq/g or more, and is even more preferably 6.5 meq/g or less, even more preferably 5.0 meq/g or less, and even more preferably 4.5 meq/g or less. The weight average molecular weight is still more preferably 200,000 or more, even more preferably 500,000 or more, and even more preferably 1,000,000 or more, and is preferably 5,000,000 or less, more preferably 3,000,000 or less, and still more preferably 1,500,000 or less.

The cationic charge density of the vinylimidazolium trichloride-vinylpyrrolidone copolymer (polyquaternium-16) is even more preferably 2.0 meq/g or more, and is even more preferably 6.5 meq/g or less, even more preferably 5.0 meq/g or less, and even even more preferably 4.5 meq/g or less. The weight average molecular weight is still more preferably 20,000 or more, and even more preferably 50,000 or more, and is still more preferably 150,000 or less.

Commercially available polymers can also be used as the component (A). Specific examples thereof include "MERQUAT 2001 Polymer" (acrylic acid-methyl acrylate-methacrylamidopropyltrimethylammonium chloride copolymer; polyquaternium-47, cationic charge density: 3.21 meq/g, Mw: 1,300,000), "MERQUAT 100" (dimethyldiallylammonium chloride polymer; polyquaternium-6, cationic charge density: 6.18 meq/g, Mw: 150,000), "MERQUAT 106" (dimethyldiallylammonium chloride polymer; polyquaternium-6, cationic charge density: 6.18 meq/g, Mw: 15,000), "MERQUAT 3940 POLYMER" (acrylamide-acrylic acid-dimethyl diallyl ammonium chloride polymer liquid; polyquaternium-39, cationic charge density: 2.97 meq/g, Mw: 150,000), "MERQUAT 280" (dimethyldiallylammonium chloride-acrylic acid copolymer liquid; polyquaternium-22, cationic charge density: 4.99 meq/g, Mw: 450,000), "MERQUAT 295" (dimethyldiallylammonium chloride-acrylic acid copolymer liquid; polyquaternium-22, cationic charge density: 6.04 meq/g, Mw: 190,000), "MERQUAT 2003PR Polymer" (acrylic acid-acrylamide-methacrylamidopropyltrimethyl ammonium chloride copolymer; polyquaternium-53), "MERQUAT 550" (diallyldimethylammonium chloride-acrylamide copolymer; polyquaternium-7), "MERQUAT 740" (diallyldimethylammonium chloride-acrylamide copolymer; polyquaternium-7, cationic charge density: 2.59 meq/g, Mw: 120,000) manufactured by Lubrizol Advanced Materials, Inc., "Luviquat FC 550" (vinylimidazolium trichloride-vinylpyrrolidone copolymer; polyquaternium-16, cationic charge density: 3.91 meq/g, Mw: 80,000), "Luviquat Excellence" (vinylimidazolium trichloride-vinylpyrrolidone copolymer; polyquaternium-16, cationic charge density: 6.65 meq/g, Mw: 40,000), "Luviquat FC 370" (vinylimidazolium trichloride-vinylpyrrolidone copolymer; polyquaternium-16, cationic charge density: 2.48 meq/g, Mw: 100,000) manufactured by BASF SE, "POIZ C-60H" manufactured by Kao Corporation (vinylimidazolium trichloride-vinylpyrrolidone copolymer; polyquaternium-16, cationic charge density: 1.5 meq/g, Mw: 600,000), "KP Polymer E" manufactured by Kao Corporation (methacryloyl ethyl trimethyl ammonium chloride copolymer (polyquaternium-37), cationic charge density: 4.8 meq/g, Mw: 300,000), "Plascize L-440" and "Plascize L-440W", Ltd. (methacryloyl ethyl dimethyl betaine-methacryloyl ethyl trimethyl ammonium chloride-methoxypolyethylene glycol methacrylate copolymer; polyquaternium-49), and "Plascize L-450" and "Plascize L-450W" (methacryloyl ethyl dimethyl betaine-methacryloyl ethyl trimethyl ammonium chloride-2-hydroxyethyl methacrylate copolymer; polyquaternium-48) manufactured by Goo Chemical Co..

### (Component (B): Anionic Polymer)

The component (B) is one or more anionic polymers selected from the group consisting of polyacrylic acid, (meth)acrylic acid/alkyl (meth)acrylate copolymers, and salts thereof, and is a polymer capable of forming a polyion complex by interaction with the component (A). In the component (B), at least a part of the carboxy group which is an anionic group may be neutralized to be in a salt state.

The anionic charge density of the component (B) is preferably 0.1 meq/g or more, more preferably 0.5 meq/g or more, still more preferably 1.0 meq/g or more, and even more preferably 2.0 meq/g or more, from the viewpoint of facilitating the formation of the polyion complex by interaction with the component (A), and is preferably 30 meq/g or less, more preferably 20 meq/g or less, and still more preferably 15 meq/g or less, from the viewpoint of stability of the composition. Thus, the anionic charge density of the component (B) is preferably 0.1 meq/g or more and 30 meq/g or less, more preferably 0.5 meq/g or more and 30 meq/g or less, still more preferably 1.0 meq/g or more and 20 meq/g or less, and even more preferably 2.0 meq/g or more and 15 meq/g or less.

The anionic charge density of the component (B) can be calculated from (the number of moles of anionic groups contained per 1 g of polymer) × 1000 (meq/g). When at least a part of the anionic groups of the component (B) is in the form of a neutralized salt, the number of moles of the anionic groups includes the number of moles of the anionic groups in the form of a salt.

The hair cosmetic composition may use two or more polymers as the component (B), and in this case, the anionic charge density of the component (B) is obtained by calculating the weighted average from the anionic charge density and the blending amount of each polymer.

The weight average molecular weight (Mw) of the component (B) is 3,000 or more, preferably 5,000 or more, and more preferably 10,000 or more, and is 50,000 or less, preferably 40,000 or less, and more preferably 30,000 or less from the viewpoints of facilitating the formation of the polyion complex, stability of the composition, and improvement in feel at the time of application to the hair and suppression of spread of the hair under high humidity conditions when the composition is used as a hair cosmetic composition. Thus, the weight average molecular weight (Mw) of the component (B) is 3,000 or more and 50,000 or less, preferably 5,000 or more and 40,000 or less, and more preferably 10,000 or more and 30,000 or less. The weight average molecular weight of the component (B) can be measured by gel permeation chromatography (GPC).

In addition, the content of the polymer having a molecular weight of 200,000 or more, which is obtained from a molecular weight distribution curve in GPC measurement, in the component (B) is preferably 2% by mass or less, more preferably 1.5% by mass or less, still more preferably 1.5% by mass or less, and even more preferably substantially 0% by mass, from the viewpoints of facilitating the formation of the polyion complex, stability of the composition, improvement in feel at the time of application to the hair and suppression of spread of the hair under high humidity conditions when the composition is used as a hair cosmetic composition.

Examples of the (meth)acrylic acid/alkyl (meth)acrylate copolymer used as the component (B) include a copolymer of (meth)acrylic acid and alkyl (meth)acrylate having an alkyl group having 1 or more and 24 or less, preferably 8 or more and 22 or less carbon atoms, such as an (acrylic acid/octyl acrylate) copolymer and an (acrylic acid/stearyl acrylate) copolymer.

The component (B) can be used alone or in combination of two or more thereof. Among the above, the component (B) is preferably one or more selected from the group consisting of a polyacrylic acid, an (acrylic acid/stearyl acrylate) copolymer, or a salt thereof, from the viewpoints of facilitating the formation of the polyion complex, stability of the composition, improvement in feel at the time of application to the hair, and suppression of spread of the hair under high humidity conditions.

Commercially available polymers can also be used as the component (B). Specific examples thereof include "ACUSOL 445G Polymer" (sodium polyacrylate) manufactured by The Dow Chemical Company, "SOFCARE SA-37W" ((acrylic acid/stearyl acrylate) copolymer) manufactured by Kao Corporation, "Diahold" ((meth)acrylic acid/alkyl (meth)acrylate copolymer) manufactured by Mitsubishi Chemical Corporation, and the like.

### (Component (C): One or more modified silicones selected from the group consisting of amino-modified silicones and aminopolyether-modified silicones)

The component (C) is one or more modified silicones selected from the group consisting of amino-modified silicones (hereinafter also referred to as "component (C1)") and aminopolyether-modified silicones (hereinafter also referred to as "component (C2)"). It is considered that by the hair cosmetic composition containing the component (C), the effect of suppressing the spread of the hair under high humidity conditions can be further improved.

### <Component (C1): Amino-Modified Silicone>

In the description herein, the amino-modified silicone means a silicone having an amino group and not having a polyether structure. The component (C1) includes, for example, an amino-modified silicone represented by the following general formula (I). 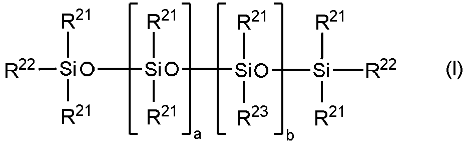 [In the formula, each R²¹ independently represents a methyl group or a hydroxy group, and each R²² independently represents an alkyl group having 1 or more and 30 or less carbon atoms, a hydroxy group, or R²³. R²³ represents a monovalent group represented by -R²⁴-Z¹ (R²⁴ represents a single bond or a divalent hydrocarbon group having 1 or more and 20 or less carbon atoms, and Z¹ represents a primary to tertiary amino group-containing group or an ammonium group-containing group). a represents a number of 0 or more and 3,000 or less, and b represents a number of 1 or more and 3,000 or less.]

In the general formula (I), R²¹ is preferably a methyl group, and R²² is preferably a methyl group or R²³.

R²³ is a monovalent group represented by -R²⁴-Z¹, and R²⁴ is preferably a divalent hydrocarbon group having 1 or more and 20 or less carbon atoms, more preferably an alkylene group having 1 or more and 20 or less carbon atoms, still more preferably a linear or branched alkylene group having 1 or more and 6 or less carbon atoms, even more preferably a methylene group, an ethylene group, a trimethylene group, a propylene group, a tetramethylene group, or a hexamethylene group, and even more preferably a trimethylene group or a propylene group.

Z¹ is a primary to tertiary amino group-containing group or an ammonium group-containing group, and is preferably an amino group-containing group represented by -N(R²⁵)₂, -NR²⁵(CH₂)_{c}N(R²⁶)₂, or -NR²⁵(CH₂)_{c}N(R²⁶)CO-R²⁷. Here, R²⁵ and R²⁶ each independently represent a hydrogen atom or an alkyl group having 1 or more and 3 or less carbon atoms, and are preferably a hydrogen atom or a methyl group. R²⁷ represents an alkyl group having 1 or more and 3 or less carbon atoms. c represents a number of 1 or more and 6 or less, and preferably a number of 2 or more and 4 or less.

In the general formula (I), R²³ is preferably -(CH₂)₃-NH₂, -(CH₂)₃-N(CH₃)₂, -(CH₂)₃-NH-(CH₂)₂-NH₂, or -(CH₂)₂-NH-(CH₂)₂-N(CH₃)₂, and more preferably -(CH₂)₃-NH₂ or - (CH₂)₃-NH-(CH₂)₂-NH₂.

Examples of the amino-modified silicone represented by the general formula (I) include one or more selected from the group consisting of amodimethicone, aminopropyl dimethicone, bis(aminopropyl)dimethicone, and bis(cetearyl)amodimethicone. More specifically, these are amino-modified silicones represented by any one of the following general formulae (Ia) to (Id). 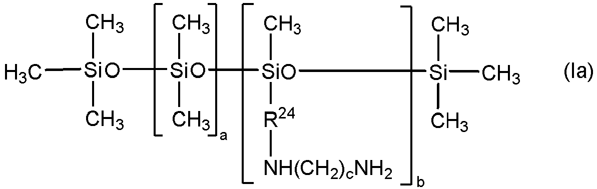 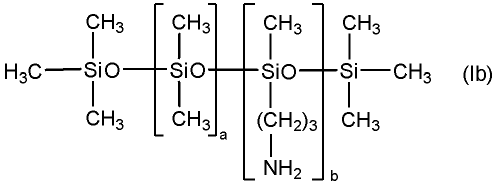 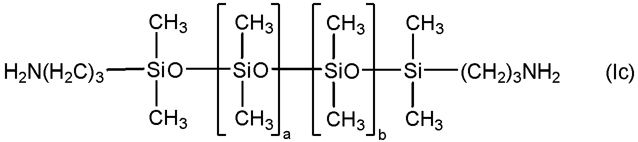 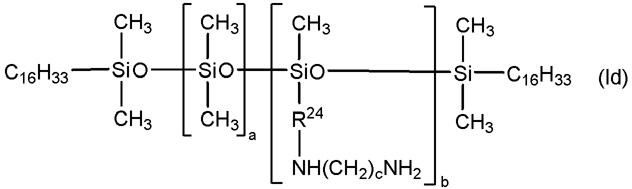 [In the formulae, a, b, c, and R²⁴ are the same as described above.]

One, or two or more of the component (C 1) can be used. From the viewpoint of further improving the effect of suppressing the spread of the hair under high humidity conditions, as the component (C1), an amino-modified silicone (amodimethicone) represented by the general formula (Ia) is more preferable.

The kinematic viscosity at 25°C of the component (C1) is preferably 20 mm²/s or more, more preferably 100 mm²/s or more, and still more preferably 500 mm²/s or more, and is preferably 10,000 mm²/s or less, more preferably 5,000 mm²/s or less, and still more preferably 2,000 mm²/s or less, from the viewpoint of further improving the effect of suppressing the spread of the hair under high humidity conditions. Thus, the kinematic viscosity at 25°C of the component (C1) is preferably 20 mm²/s or more and 10,000 mm²/s or less, more preferably 100 mm²/s or more and 5,000 mm²/s or less, and still more preferably 500 mm²/s or more and 2,000 mm²/s or less.

The kinematic viscosity of the component (C1) is a value measured in accordance with "Methods for viscosity measurement of liquid" specified in JIS Z 8803:2011 or ASTM D 445-46T at 25°C, and can be measured using, for example, an Ubbelohde's viscometer.

Commercially available amino-modified silicones can also be used as the component (C1). Examples of the amino-modified silicone (amodimethicone) represented by the general formula (Ia) include "Silicone SF 8457 C" manufactured by Dow Toray Co., Ltd.

### <Component (C2): Aminopolyether-Modified Silicone>

In the description herein, the aminopolyether-modified silicone means a silicone having an amino group and a polyether structure.

The polyether structure in the component (C2) is preferably a polyoxyalkylene group from the viewpoint of the effect of suppressing the spread of the hair under high humidity conditions and from the viewpoint of the availability.

From the viewpoint of further improving the effect of suppressing the spread of the hair under high humidity conditions, in a case where the number of moles of silicon atoms in the component (C2) is Si and the average addition mole number of alkylene oxides is AO, Si/AO is preferably 0.05 or more, more preferably 0.1 or more, still more preferably 0.3 or more, even more preferably 0.5 or more, and even more preferably 0.7 or more, and is preferably 3.0 or less, more preferably 2.8 or less, still more preferably 2.6 or less, and even more preferably 2.4 or less. Thus, the Si/AO of the component (C2) is preferably 0.05 or more and 3.0 or less, more preferably 0.1 or more and 2.8 or less, still more preferably 0.3 or more and 2.8 or less, even more preferably 0.5 or more and 2.6 or less, and even more preferably 0.7 or more and 2.4 or less.

The Si/AO can be calculated from the integral value of H of the silicon-bonded hydrogen atom and hydrocarbon group and the integral value of H of the oxyalkylene group, which are measured by ¹H-NMR measurement, and can be specifically measured by the method described in Examples.

From the viewpoint of further improving the effect of suppressing the spread of the hair under high humidity conditions, the nitrogen content of the component (C2) is preferably 0.1% by mass or more, more preferably 0.2% by mass or more, still more preferably 0.5% by mass or more, and even more preferably 1.0% by mass or more, and is preferably 2.5% by mass or less, more preferably 2.0% by mass or less, still more preferably 1.8% by mass or less, and even more preferably 1.5% by mass or less. Thus, the nitrogen content of the component (C2) is preferably 0.1% by mass or more and 2.5% by mass or less, more preferably 0.2% by mass or more and 2.0% by mass or less, still more preferably 0.5% by mass or more and 1.8% by mass or less, and even more preferably 1.0% by mass or more and 1.5% by mass or less. The nitrogen content of the component (C2) is a value measured in accordance with the potentiometric titration method specified in JIS K 0113:2005.

The component (C2) is preferably an aminopolyether-modified silicone represented by the following general formula (II). 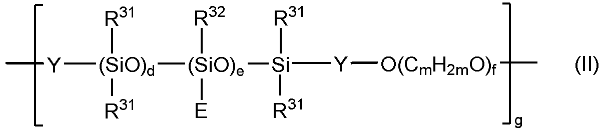 [In the formula (II), R³¹ represents a hydrogen atom or a monovalent hydrocarbon group having 1 or more and 6 or less carbon atoms. R³² represents either R³¹ or E. E represents a monovalent group represented by -R³³-Z² (R³³ represents a single bond or a divalent hydrocarbon group having 1 or more and 20 or less carbon atoms, and Z² represents a primary to tertiary amino group-containing group or an ammonium group-containing group). Y represents an alkylene group having 1 or more and 6 or less carbon atoms.
d represents a number of 2 or more, e represents a number of 1 or more, f represents a number of 2 or more and 100 or less, and g represents a number of 1 or more.
   m represents a number of 2 or more and 10 or less. The bonding order of the structural units in the parentheses is not limited, and the bonding form may be a block form or a random form. The f number of CₘH₂ₘO may be the same or different. In addition, a plurality of R³¹, R³², E, and Y may be the same or different.]

When the component (C2) is the aminopolyether-modified silicone represented by the general formula (II), the Si/AO is represented by (d + e + 1)/f

In the general formula (II), R³¹ is a hydrogen atom or a monovalent hydrocarbon group having 1 or more and 6 or less carbon atoms, preferably a hydrogen atom, an alkyl group having 1 or more and 6 or less carbon atoms, or a phenyl group, more preferably a methyl group or an ethyl group, and still more preferably a methyl group.

In the general formula (II), R³² represents either R³¹ or E. E represents a monovalent group represented by -R³³-Z² (R³³ represents a single bond or a divalent hydrocarbon group having 1 or more and 20 or less carbon atoms).

R³³ is preferably a divalent hydrocarbon group having 1 or more and 20 or less carbon atoms, more preferably an alkylene group having 1 or more and 20 or less carbon atoms, still more preferably a linear or branched alkylene group having 1 or more and 6 or less carbon atoms, even more preferably a methylene group, an ethylene group, a trimethylene group, a propylene group, a tetramethylene group, or a hexamethylene group, and even more preferably a trimethylene group or a propylene group.

Z² is a primary to tertiary amino group-containing group or an ammonium group-containing group, and is preferably an amino group-containing group represented by -N(R³⁴)₂, -NR³⁴(CH₂)ₕN(R³⁴)₂, or -NR³⁴(CH₂)ₕN(R³⁵)CO-R³⁶. Here, R³⁴ and R³⁵ each independently represent a hydrogen atom or an alkyl group having 1 or more and 3 or less carbon atoms, and are preferably a hydrogen atom or a methyl group. R³⁶ represents an alkyl group having 1 or more and 3 or less carbon atoms. h represents a number of 1 or more and 6 or less, and preferably a number of 2 or more and 4 or less.

In the general formula (II), E is preferably -(CH₂)₃-NH₂, -(CH₂)₃-N(CH₃)₂, -(CH₂)₃-NH-(CH₂)₂-NH₂, or -(CH₂)₂-NH-(CH₂)₂-N(CH₃)₂, and more preferably -(CH₂)₃-NH-(CH₂)₂-NH₂.

In the general formula (II), Y is an alkylene group having 1 or more and 6 or less carbon atoms, and is preferably an ethylene group, a propylene group, a trimethylene group, a n-butylene group (tetramethylene group), or an i-butylene group, and more preferably a n-butylene group or an i-butylene group. The i-butylene group referred to herein includes - CH(CH₃)CH₂CH₂-, -CH₂CH(CH₃)CH₂-, and -CH₂CH₂CH(CH₃)-.

In the general formula (II), d represents a number of 2 or more, e represents a number of 1 or more, f represents a number of 2 or more and 100 or less, and g represents a number of 1 or more. d is preferably a number of 2 or more and 1000 or less, and more preferably a number of 2 or more and 100 or less. e is preferably a number of 1 or more and 50 or less, f is preferably a number of 4 or more and 50 or less, and more preferably a number of 10 or more and 18 or less, and g is preferably a number of 1 or more and 100 or less.

In the general formula (II), m represents a number of 2 or more and 10 or less, preferably a number of 2 or more and 6 or less, and more preferably a number of 2 or more and 4 or less.

The component (C2) is more preferably an aminopolyether-modified silicone represented by the following general formula (IIa). 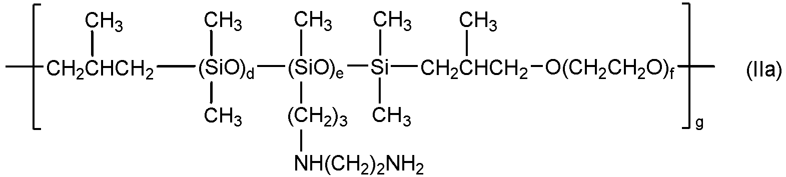 [In the formula (IIa), d to g are the same as described above.]

The kinematic viscosity at 25°C of the component (C2) is preferably 5,000 mm²/s or more, more preferably 20,000 mm²/s or more, and still more preferably 40,000 mm²/s or more, and is preferably 200,000 mm²/s or less, more preferably 150,000 mm²/s or less, and still more preferably 100,000 mm²/s or less, from the viewpoint of further improving the effect of suppressing the spread of the hair under high humidity conditions. Thus, the kinematic viscosity at 25°C of the component (C2) is preferably 5,000 mm²/s or more and 200,000 mm²/s or less, more preferably 20,000 mm²/s or more and 150,000 mm²/s or less, and still more preferably 40,000 mm²/s or more and 100,000 mm²/s or less.

The kinematic viscosity of the component (C2) can be measured by the same method as that for the component (C1).

Commercially available aminopolyether-modified silicones can also be used as the component (C2). Examples of the aminopolyether-modified silicone represented by the general formula (IIa) include "DOWSIL SS-3588 FLUID" ((bis-isobutyl-PEG-15/amodimethicone) copolymer, Si/AO = 2.39, nitrogen content: 1.0% by mass, active ingredient amount: 71.5% by mass), "DOWSIL SILSTYLE 104" ((bis-isobutyl-PEG-14/amodimethicone) copolymer, nitrogen content: 1.2% by mass, Si/AO = 2.46), and "DOWSIL SILSTYLE 201" ((bis-isobutyl-PEG-14/amodimethicone) copolymer, nitrogen content: 1.2% by mass, Si/AO = 0.80), all manufactured by Dow Toray Co., Ltd.

One, or two or more of the component (C) can be used.

Among the above, the component (C) is preferably one or more selected from the group consisting of the amino-modified silicone represented by the general formula (I) and the aminopolyether-modified silicone represented by the general formula (II), and more preferably one or more selected from the group consisting of the amino-modified silicone represented by the general formula (Ia) and the aminopolyether-modified silicone represented by the general formula (IIa), from the viewpoint of further improving the effect of suppressing the spread of the hair under high humidity conditions.

Optional components other than the components (A) to (C) can also be blended in the hair cosmetic composition. From the viewpoint of facilitating the formation of the polyion complex by dispersing the component (A) and the component (B), and from the viewpoint of obtaining a stable emulsion composition, the hair cosmetic composition preferably further contains an organic acid and water.

In addition, in a case where the hair cosmetic composition is a hair rinse, a hair conditioner, a hair treatment, or a hair styling agent, from the viewpoint of further improving the feel at the time of application to the hair and the feel of the hair after treatment, it is preferable that the hair cosmetic composition contains one or more selected from the group consisting of a cationic surfactant, a higher alcohol, a non-aromatic polyol, and an oil agent, and it is more preferable that the hair cosmetic composition contains all of these.

### (Organic Acid)

The organic acid contributes to stably forming a polyion complex in the hair cosmetic composition.

Examples of the organic acid include a carboxylic acid-based compound and a sulfonic acid-based compound other than the component (B), and a compound having a molecular weight of preferably 500 or less and more preferably 200 or less is used.

Examples of the carboxylic acid-based compound include aliphatic monocarboxylic acids having 4 or less carbon atoms such as acetic acid, propionic acid, and butanoic acid; aromatic monocarboxylic acids such as benzoic acid; aliphatic dicarboxylic acids such as malonic acid, succinic acid, glutaric acid, adipic acid, maleic acid, and fumaric acid; aromatic dicarboxylic acids such as phthalic acid and isophthalic acid; polycarboxylic acids such as polyglutamic acid; hydroxycarboxylic acids such as lactic acid, malic acid, glycolic acid, hydroxyacrylic acid, glyceric acid, tartaric acid; and citric acid; and acidic amino acids such as glutamic acid and aspartic acid; and one, or two or more of these can be used.

Examples of the sulfonic acid-based compound include aliphatic sulfonic acids such as methanesulfonic acid and ethanesulfonic acid; and aromatic sulfonic acids such as p-toluenesulfonic acid and naphthalenesulfonic acid, and one, or two or more of these can be used.

From the viewpoint of stably forming a polyion complex to form a highly stable hair cosmetic composition and from the viewpoint of being used in a hair cosmetic composition, the organic acid is preferably one or more selected from the group consisting of aliphatic dicarboxylic acids, hydroxycarboxylic acids, and aromatic sulfonic acids, more preferably one or more selected from the group consisting of succinic acid, lactic acid, malic acid, glycolic acid, p-toluenesulfonic acid, and naphthalenesulfonic acid, still more preferably one or more selected from the group consisting of succinic acid and lactic acid, and even more preferably lactic acid.

At least a part of the organic acid may be in a state of an organic acid salt at the time of blending. As the salt of the organic acid, an alkali metal salt or an alkali metal salt of the organic acid is preferable, an alkali metal salt is more preferable, and one or more selected from the group consisting of a sodium salt and a potassium salt is still more preferable, and a sodium salt is even more preferable, from the viewpoint of using in a hair cosmetic composition or the like, and from the viewpoint of easiness of availability.

The proportion of the organic acid salt in the total amount of the organic acid and the organic acid salt is preferably 50% by mass or less, more preferably 20% by mass or less, and still more preferably 5% by mass or less, and may be 0% by mass, from the viewpoints of facilitating the formation of the polyion complex, stability of the composition, improvement in feel at the time of application to the hair, and suppression of spread of the hair under high humidity conditions. The proportion (% by mass) of the organic acid salt herein means % by mass converted to an organic acid.

### (Water)

As water used in the hair cosmetic composition, deionized water or distilled water is preferred. Tap water, ground water, or the like sterilized with hypochlorous acid or the like may be used within a range that does not impair the stability of the composition.

### (Component (D): Cationic Surfactant)

The hair cosmetic composition preferably further contains a cationic surfactant as component (D), from the viewpoint of further improving the feel at the time of application to the hair and the feel of the hair after treatment.

Examples of the cationic surfactant include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof.

Examples of the alkyltrimethylammonium salt (i) includes an alkyltrimethylammonium salt having an alkyl group having preferably 12 or more and 22 or less carbon atoms, and more preferably 16 or more and 20 or less carbon atoms, and specific examples thereof include cetyltrimethylammonium chloride (cetrimonium chloride), stearyltrimethylammonium chloride (steartrimonium chloride), and behenyltrimethylammonium chloride (behentrimonium chloride).

Examples of the alkoxyalkyltrimethylammonium salt (ii) includes an alkoxyalkyltrimethylammonium salt having an alkoxy group having preferably 12 or more and 22 or less carbon atoms, and more preferably 16 or more and 20 or less carbon atoms, and specific examples thereof include stearoxypropyltrimethylammonium chloride, stearoxyethyltrimethylammonium chloride, and stearoxyhydroxypropyltrimethylammonium chloride.

Examples of the dialkyldimethylammonium salt (iii) includes a dialkyldimethylammonium salt having an alkyl group having preferably 12 or more and 22 or less carbon atoms, and more preferably 16 or more and 20 or less carbon atoms, and specific examples thereof include distearyldimethylammonium chloride.

Examples of the alkylamidoalkyltrimethylammonium salt (iv) includes an alkylamidoalkyltrimethylammonium salt in which the alkyl carbon number of the alkylamido moiety is preferably 11 or more and 21 or less, and more preferably 13 or more and 19 or less, and specific examples thereof include palmitamidopropyltrimethylammonium chloride (palmitamidopropyltrimonium chloride).

The alkyldimethylamine (v), the alkoxyalkyldimethylamine (vi), and the alkylamidoalkyldimethylamine (vii) each react with an acid to become a tertiary amine salt, and become a cationic surfactant.

The alkyl group in the alkyldimethylamine and a salt thereof (v), and the alkoxy group in the alkoxyalkyldimethylamine and a salt thereof (vi) each has preferably 12 or more and 22 or less carbon atoms, and more preferably 16 or more and 20 or less carbon atoms.

The alkyl carbon number of the alkylamide moiety in the alkylamidoalkyldimethylamine and a salt thereof (vii) is preferably 11 or more and 21 or less, and more preferably 15 or more and 19 or less.

The amines (v) to (vii) may be reacted with an acid in advance and then blended into the hair cosmetic composition as a salt, or may be blended into the hair cosmetic composition as an amine and then an acid may be blended into the hair cosmetic composition to form a salt in the composition. Accordingly, the amines and their salts are defined herein as cationic surfactants. The content or the blending amount of the amine is converted in terms of the mass of the amine

Examples of the salt of the amines (v) to (vii) include salts with an organic acid or an inorganic acid. Examples of the organic acid include monocarboxylic acids such as acetic acid and propionic acid; dicarboxylic acids such as malonic acid, succinic acid, glutaric acid, adipic acid, maleic acid, fumaric acid, and phthalic acid; polycarboxylic acids such as polyglutamic acid; hydroxycarboxylic acids such as glycolic acid, lactic acid, hydroxyacrylic acid, glyceric acid, malic acid, tartaric acid, and citric acid; and acidic amino acids such as glutamic acid and aspartic acid. Examples of the inorganic acid include hydrochloric acid, sulfuric acid, and phosphoric acid. Among these, organic acids are preferable, and one or more selected from the group consisting of dicarboxylic acids, hydroxycarboxylic acids, and acidic amino acids are more preferable. The dicarboxylic acid is more preferably one or more selected from the group consisting of maleic acid and succinic acid. The hydroxycarboxylic acid is more preferably one or more selected from the group consisting of glycolic acid, lactic acid, and malic acid. As the acidic amino acid, glutamic acid is more preferable.

Examples of the alkyldimethylamine and a salt thereof (v) include N,N-dimethylbehenylamine, N,N-dimethylstearylamine, and organic acid salts thereof, and a lactic acid salt of N,N-dimethylbehenylamine and a glycolic acid salt of N,N-dimethylstearylamine are preferable.

Examples of the alkoxyalkyldimethylamine and a salt thereof (vi) include N,N-dimethyl-3-hexadecyloxypropylamine, N,N-dimethyl-3-octadecyloxypropylamine (stearoxypropyldimethylamine), and organic acid salts thereof, and N,N-dimethyl-3-hexadecyloxypropylamine or a lactic acid salt thereof, and N,N-dimethyl-3-octadecyloxypropylamine (stearoxypropyldimethylamine) or a lactic acid salt thereof are preferable.

Examples of the alkylamidoalkyldimethylamine and a salt thereof (vii) include N-[3-(dimethylamino)propyl]docosanamide and N-[3-(dimethylamino)propyl]stearamide, and organic acid salts thereof, and among these, a lactic acid salt of N-[3-(dimethylamino)propyl]docosanamide and a glycolic acid salt of N-[3-(dimethylamino)propyl]stearamide are preferable.

One, or two or more of the component (D) can be used.

Among the above, from the viewpoint of further improving the feel at the time of application to the hair and the feel of the hair after treatment, the component (D) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof, more preferably one or more selected from the group consisting of (i) an alkyltrimethylammonium salt, (ii) an alkoxyalkyltrimethylammonium, and (v) an alkyldimethylamine and a salt thereof, still more preferably one or more selected from the group consisting of behenyltrimethylammonium chloride, stearoxypropyltrimethylammonium chloride, stearoxyethyltrimethylammonium chloride, stearoxyhydroxypropyltrimethylammonium chloride, N,N-dimethyl-3-hexadecyloxypropylamine or a lactic acid salt thereof, and N,N-dimethyl-3-octadecyloxypropylamine (stearoxypropyldimethylamine) or a lactic acid salt thereof, and even more preferably one or more selected from the group consisting of N,N-dimethyl-3-octadecyloxypropylamine (stearoxypropyldimethylamine) or a lactic acid salt thereof.

### (Component (E): Higher Alcohol)

The hair cosmetic composition preferably further contains a higher alcohol as component (E), from the viewpoint of further improving the feel at the time of application to the hair and the feel of the hair after treatment.

Examples of the higher alcohol include aliphatic monohydric alcohols having 12 or more carbon atoms, preferably 12 or more and 22 or less carbon atoms. The higher alcohol may be one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol.

Among the above, from the viewpoint of further improving the feel at the time of application to the hair and the feel of the hair after treatment, one or more selected from the group consisting of cetyl alcohol, myristyl alcohol, stearyl alcohol, and behenyl alcohol are preferable, and stearyl alcohol is more preferable.

### (Component (F): Non-Aromatic Polyol)

The hair cosmetic composition preferably further contains a non-aromatic polyol as component (F) from the viewpoint of further improving the feel at the time of application to the hair and the feel of the hair after treatment, and from the viewpoint of improving the moisture retention.

Examples of the non-aromatic polyol used as the component (F) include low molecular weight diols and triols having 6 or less carbon atoms, such as 1,3-butylene glycol, glycerin, ethylene glycol, propylene glycol, and dipropylene glycol. One, or two or more of these can be used.

Among the above, from the viewpoint of further improving the feel at the time of application to the hair and the feel of the hair after the treatment, and from the viewpoint of improving the moisture retention, one or more selected from the group consisting of 1,3-butylene glycol and dipropylene glycol are preferable, and dipropylene glycol is more preferable.

### (Component (G): Oil Agent)

The hair cosmetic composition preferably further contains, as component (G), an oil agent other than the component (C) and the component (E), from the viewpoint of further improving the feel at the time of application to the hair and the feel of the hair after treatment.

Examples of the oil agent include (a) silicone oil, (b) ester oil, (c) ether oil, (d) hydrocarbon oil, (e) higher fatty acid, and (f) wax, and one, or two or more of these can be used.

As the silicone oil (a), one or more selected from the group consisting of dimethylpolysiloxane (dimethicone), dimethiconol (dimethylpolysiloxane having a hydroxy group at the terminal), methylphenylpolysiloxane, and modified silicone other than the component (C) are preferable.

Examples of the modified silicone other than the component (C) include polyether-modified silicone, glyceryl-modified silicone, carboxy-modified silicone, fatty acid-modified silicone, alcohol-modified silicone, aliphatic alcohol-modified silicone, epoxy-modified silicone, fluorine-modified silicone, and alkyl-modified silicone.

Among the above, from the viewpoint of further improving the feel at the time of application to the hair and the feel of the hair after treatment, as the silicone oil (a), one or more selected from the group consisting of dimethylpolysiloxane, dimethiconol, methylphenylpolysiloxane, and modified silicones other than the component (C) are preferable, one or more selected from the group consisting of dimethylpolysiloxane, dimethiconol, and polyether-modified silicones are more preferable, and dimethylpolysiloxane is still more preferable.

Examples of the ester oil (b) include synthetic ester oils and natural oils and fats, and examples thereof include esters of a monovalent carboxylic acid and a monohydric alcohol, esters of a monovalent carboxylic acid and a polyhydric alcohol, and esters of a polyvalent carboxylic acid and a monohydric alcohol.

Examples of the ester of a monovalent carboxylic acid and a monovalent alcohol include an ester represented by the following general formula (1).

R¹-COO-R² (1)

In the general formula (1), R¹ represents a linear or branched alkyl group or alkenyl group having 1 or more and 25 or less carbon atoms, or an aromatic-containing hydrocarbon group having 6 or more and 24 or less carbon atoms, which may be substituted with a hydroxy group, and R² represents a linear or branched alkyl group or alkenyl group having 1 or more and 30 or less carbon atoms.

When R¹ is an alkyl group or an alkenyl group, the number of carbon atoms is preferably 7 or more, and is preferably 23 or less, more preferably 21 or less, still more preferably 19 or less, and even more preferably 17 or less.

When R¹ is an aromatic-containing hydrocarbon group, the number of carbon atoms is preferably 6 or more, and is preferably 22 or less, and more preferably 20 or less.

The number of carbon atoms of R² is preferably 2 or more, and is preferably 28 or less, more preferably 24 or less, and still more preferably 20 or less.

Even more preferably, in the ester represented by the general formula (1), R¹ is a linear or branched alkyl group having 7 or more and 17 or less carbon atoms, and R² is a linear or branched alkyl group having 2 or more and 24 or less carbon atoms.

Specific examples of the ester represented by the general formula (1) include one or more selected from the group consisting of cetyl isooctanoate, stearyl isooctanoate, isononyl isononanoate, isotridecyl isononanoate, hexyl laurate, isostearyl laurate, butyl myristate, isopropyl myristate, decyl myristate, isotridecyl myristate, isocetyl myristate, isostearyl myristate, 2-octyldodecyl myristate, isopropyl palmitate, 2-ethylhexyl palmitate, isostearyl palmitate, 2-hexyldecyl palmitate, 2-ethylhexyl stearate, 2-hexyldecyl stearate, isopropyl isostearate, 2-hexyldecyl isostearate, ethyl oleate, isodecyl oleate, oleyl oleate, octyldodecyl oleate, ethyl linoleate, isopropyl linoleate, lanolin acetate, methyl castor oil fatty acid (methyl ricinoleate), and alkyl benzoate (alkyl having 12 to 15 carbon atoms).

Examples of the ester of a monovalent carboxylic acid and a monovalent alcohol also include an ester represented by the following general formula (2).

R³-COO-(AO)ₙ-R⁴ (2)

In the general formula (2), R³ represents a linear or branched alkyl group or alkenyl group having 1 or more and 25 or less carbon atoms, which may be substituted with a hydroxy group, and R⁴ represents an aromatic-containing hydrocarbon group having 6 or more and 24 or less carbon atoms. AO represents an alkyleneoxy group having 2 or more and 4 or less carbon atoms, and n represents an average addition mole number of 1 or more and 50 or less.

R³ is preferably an alkyl group having 7 or more carbon atoms, and having preferably 23 or less, more preferably 21 or less, and still more preferably 19 or less carbon atoms.

R⁴ is preferably an aromatic-containing hydrocarbon group having 6 or more carbon atoms, and having preferably 22 or less, more preferably 20 or less, and still more preferably 18 or less carbon atoms, and even more preferably a benzyl group.

The AO group is preferably a propyleneoxy group, and n is preferably 1 or more and 10 or less, and more preferably 1 or more and 5 or less.

Specific examples of the ester represented by the general formula (2) include an ester of myristic acid with 3 mol propylene oxide adduct of benzyl alcohol ("Crodamol STS" manufactured by Croda International PLC), and an ester of 2-ethylhexanoic acid with 3 mol propylene oxide adduct of benzyl alcohol ("Crodamol SFX" manufactured by Croda International PLC).

Examples of the ester of a monovalent carboxylic acid and a monovalent alcohol include an ester represented by the following general formula (3).

R⁵-(OCOR⁶)ₚ (3)

In the general formula (3), R⁵ represents a polyhydric alcohol residue, and preferably a hydrocarbon group having 2 or more and 10 or less carbon atoms, R⁶ represents a monovalent carboxylic acid residue having 1 or more and 25 or less carbon atoms, and p represents an integer of 2 or more and 10 or less.

R⁵ may have an ether bond, but is preferably a linear or branched hydrocarbon group having 2 or more and 10 or less carbon atoms. The number of p is the same as the number of hydroxy groups of the polyhydric alcohol.

R⁶ is preferably an alkyl group having 7 or more carbon atoms, and from the same viewpoint as described above, is preferably an alkyl group having 23 or less carbon atoms, more preferably 21 or less carbon atoms, and still more preferably 19 or less carbon atoms.

Even more preferably, in the ester represented by the general formula (3), R⁵ is a glycerin residue or a pentaerythritol residue, and R⁶ is an alkyl group having 7 or more and 19 or less carbon atoms.

Specific examples of the ester represented by the general formula (3) include propylene glycol dicaprate, propylene glycol dioleate, neopentyl glycol dicaprate, neopentyl glycol di-2-ethylhexanoate, propanediol di(capryate/caprate), propanediol diisostearate, ethylene glycol di-2-ethylhexanoate, glyceryl tri(caprylate/caprate), glyceryl tri-2-ethylhexylate, glyceryl tri-2-heptylundecanoate, trimethylolpropane tri-2-ethylhexylate, trimethylolpropane triisostearate, pentaerythritol tetra2-ethylhexylate, and natural fats and oils.

Examples of the natural fats and oils include triglycerides such as avocado oil, olive oil, sesame oil, rice bran oil, safflower oil, soybean oil, corn oil, rapeseed oil, castor oil, cottonseed oil, and mink oil.

Examples of the ester of a polyvalent carboxylic acid and a monovalent alcohol also include an ester represented by the following general formula (4).

R⁷-(COOR⁸)_{q} (4)

In the general formula (4), R⁷ is a polyvalent carboxylic acid residue having 2 or more and 10 or less carbon atoms, R⁸ represents a monovalent alcohol residue having 1 or more and 24 or less carbon atoms, and q is an integer of 2 or more and 10 or less. Further, the number of q is the same as the number of carboxy groups of the polyvalent carboxylic acid.

R⁸ preferably has 3 or more carbon atoms, more preferably 6 or more carbon atoms, and from the same viewpoint as described above, preferably has 22 or less, more preferably 20 or less, and still more preferably 18 or less carbon atoms.

Even more preferably, in ester represented by the general formula (4), R⁷ is a trimellitic acid residue and R⁸ is a branched alkyl group having 6 or more and 18 or less carbon atoms.

Specific examples of the ester represented by the general formula (4) include diisostearyl malate, di-2-ethylhexyl succinate, diisobutyl adipate, di-2-heptylundecyl adipate, di-2-ethylhexyl sebacate, diisopropyl sebacate, and tri-2-ethylhexyl trimellitate.

Among these, from the viewpoint of further improving the feel at the time of application to the hair and the feel of the hair after treatment, esters of a monovalent carboxylic acid and a monohydric alcohol represented by the general formula (1), esters of a monovalent carboxylic acid and a polyhydric alcohol represented by the general formula (3), or esters of a polyvalent carboxylic acid and a monohydric alcohol represented by the general formula (4), are preferable, and one or more selected from the group consisting of esters of the general formula (1) in which R¹ is a linear or branched alkyl group having 7 or more and 17 or less carbon atoms, and R² is a linear or branched alkyl group having 2 or more and 24 or less carbon atoms, esters of the general formula (3) in which R⁵ is a glycerin residue and R⁶ is an alkyl group having 7 or more and 19 or less carbon atoms, and esters of the general formula (4) in which R⁷ is a trimellitic acid residue and R⁸ is a branched alkyl group having 6 or more and 18 or less carbon atoms, are more preferable.

Examples of the ether oil (c) include a dialkyl ether compound represented by the following general formula (5) or a polyoxyalkylene alkyl ether compound represented by the following general formula (6).

R⁹-O-R¹⁰ (5)

In the general formula (5), R⁹ and R¹⁰ each independently represent a linear or branched alkyl group or alkenyl group having 6 or more and 22 or less carbon atoms, or an aromatic hydrocarbon group having 6 or more and 24 or less carbon atoms.

R⁹ and R¹⁰ are each preferably an alkyl group, and the number of carbon atoms thereof is preferably 8 or more, and is preferably 18 or less, more preferably 16 or less, and still preferably 12 or less, from the viewpoint of further improving the feel at the time of application to the hair and the feel of the hair after treatment.

R¹¹-O-(PO)r(EO)s-H (6)

In the general formula (6), R¹¹ represents an alkyl group or alkenyl group having 6 or more and 22 or less carbon atoms, PO represents a propyleneoxy group, and EO represents an ethyleneoxy group. r represents an average addition mole number of 0.1 or more and 15 or less, and s represents an average addition mole number of 0 or more and 10 or less. When s is not 0, the addition form of PO and EO may be random or block.

The number of carbon atoms of R¹¹ is preferably 8 or more, and is preferably 20 or less, more preferably 18 or less, and still more preferably 12 or less.

The average addition mole number r is preferably 1 or more, more preferably 2 or more, and still more preferably 3 or more, and is preferably 13 or less, and more preferably 10 or less, from the viewpoint of further improving the feel at the time of application to the hair and the feel of the hair after treatment, and the average addition mole number s is preferably 5 or less, more preferably 1 or less, and still more preferably 0.

The polyoxyalkylene alkyl ether compound represented by the general formula (6) is preferably one or more selected from the group consisting of polypropylene glycol, polyoxypropylene octyl ether, polyoxypropylene decyl ether, and polyoxypropylene lauryl ether in which the average addition mole number r of the propyleneoxy group is 3 or more and 10 or less.

Examples of the hydrocarbon oil (d) include squalene, squalane, liquid paraffin, liquid isoparaffin, isohexadecane, isoeicosane, hydrogenated polyisobutene, light liquid isoparaffin, heavy liquid isoparaffin, α-olefin oligomer, and cycloparaffin.

Examples of the higher fatty acid (e) include fatty acids having 8 or more carbon atoms, preferably 12 or more carbon atoms, and examples thereof include capric acid, lauric acid, myristic acid, palmitic acid, stearic acid, behenic acid, oleic acid, coconut oil fatty acid, lanolin fatty acid, isostearic acid, and isopalmitic acid.

Examples of the wax (f) include beeswax, whale wax, lanolin, and carnauba wax.

As the oil agent as the component (G), one, or two or more of the above can be used. Among the above, from the viewpoint of further improving the feel at the time of application to the hair and the feel of the hair after treatment, one or more selected from the group consisting of (a) silicone oil, (b) ester oil, (d) hydrocarbon oil, and (e) higher fatty acid are preferable, those containing one or more selected from the group consisting of (a) silicone oil and (e) higher fatty acid are more preferable, those containing one or more selected from the group consisting of dimethylpolysiloxane, dimethiconol, methylphenylpolysiloxane, modified silicone other than the component (C), and fatty acids having 12 or more carbon atoms are still more preferable, those containing one or more selected from the group consisting of dimethylpolysiloxane, dimethiconol, polyether-modified silicone, lauric acid, myristic acid, palmitic acid, stearic acid, behenic acid, oleic acid, coconut oil fatty acids, lanolin fatty acids, isostearyl acid, and isopalmitic acid are even more preferable, those containing one or more selected from the group consisting of dimethylpolysiloxane and lanolin fatty acids are even more preferable, and those containing dimethylpolysiloxane and lanolin fatty acids are even more preferable.

### (Content)

The contents or blending amounts of the respective components in the hair cosmetic composition are preferably as follows from the viewpoints of stability of the composition, improvement in feel at the time of application to the hair, and suppression of spread of the hair under high humidity conditions.

The content of the component (A) in the hair cosmetic composition is preferably 0.01% by mass or more, more preferably 0.02% by mass or more, and still more preferably 0.03% by mass or more, from the viewpoint of facilitating the formation of the polyion complex, from the viewpoint of the improvement of the feel at the time of application to the hair, and from the viewpoint of the suppression of the spread of the hair under high humidity conditions. On the other hand, from the viewpoint of improving the emulsified state, the content is preferably less than 0.50% by mass, more preferably 0.30% by mass or less, and still more preferably 0.20% by mass or less. Thus, the content of the component (A) in the hair cosmetic composition is preferably 0.01% by mass or more and less than 0.50% by mass, more preferably 0.02% by mass or more and 0.30% by mass or less, and still more preferably 0.03% by mass or more and 0.20% by mass or less.

The content of the component (B) in the hair cosmetic composition is preferably 0.01% by mass or more, and more preferably 0.015% by mass or more, from the viewpoint of facilitating the formation of the polyion complex, from the viewpoint of the improvement of the feel at the time of application to the hair, and from the viewpoint of the suppression of the spread of the hair under high humidity conditions. On the other hand, from the viewpoint of improving the emulsified state, the content is preferably less than 0.50% by mass, more preferably 0.30% by mass or less, still more preferably 0.20% by mass or less, and even more preferably 0.10% by mass or less. Thus, the content of the component (B) in the hair cosmetic composition is preferably 0.01% by mass or more and less than 0.50% by mass, more preferably 0.01% by mass or more and 0.30% by mass or less, still more preferably 0.01% by mass or more and 0.20% by mass or less, and even more preferably 0.015% by mass or more and 0.10% by mass or less.

The total content of the component (A) and the component (B) in the hair cosmetic composition is less than 1.0% by mass, preferably 0.9% by mass or less, more preferably 0.5% by mass or less, and still more preferably 0.3% by mass or less, from the viewpoint of stability of the composition. On the other hand, from the viewpoint of improving the emulsified state and suppressing the spread of the hair under high humidity conditions, the total content is preferably 0.01% by mass or more, more preferably 0.03% by mass or more, and still more preferably 0.05% by mass or more. Thus, the total content of the component (A) and the component (B) in the hair cosmetic composition is less than 1.0% by mass, preferably 0.01% by mass or more and 0.9% by mass or less, more preferably 0.03% by mass or more and 0.9% by mass or less, still more preferably 0.05% by mass or more and 0.9% by mass or less, even more preferably 0.05% by mass or more and 0.5% by mass or less, and even more preferably 0.05% by mass or more and 0.3% by mass or less.

The ratio of the number of moles of cationic charges of the component (A) to the number of moles of anionic charges of the component (B) in the hair cosmetic composition is an amount of preferably 5/95 to 95/5, more preferably 10/90 to 90/10, still more preferably 20/80 to 80/20, even more preferably 20/80 to 60/40, and even more preferably 40/60 to 60/40, from the viewpoint of improving the emulsified state and from the viewpoint of improving the feel at the time of application to the hair.

The content of the component (C) in the hair cosmetic composition is 0.03% by mass or more, preferably 0.05% by mass or more, and more preferably 0.1% by mass or more, from the viewpoints of the improvement of the feel at the time of application to the hair and the suppression of the spread of the hair under high humidity conditions. On the other hand, from the viewpoint of improving the emulsified state, the content is preferably 5.0% by mass or less, more preferably 2.0% by mass or less, still more preferably 1.0% by mass or less, and even more preferably 0.5% by mass or less. Thus, the content of the component (C) in the hair cosmetic composition is 0.03% by mass or more, preferably 0.03% by mass or more and 5.0% by mass or less, more preferably 0.05% by mass or more and 5.0% by mass or less, still more preferably 0.05% by mass or more and 2.0% by mass or less, even more preferably 0.1% by mass or more and 1.0% by mass or less, and even more preferably 0.1% by mass or more and 0.5% by mass or less.

The mass ratio [{(A) + (B)}/(C)] of the total content of the component (A) and the component (B) to the content of the component (C) in the hair cosmetic composition is preferably 0.01 or more, more preferably 0.05 or more, still more preferably 0.10 or more, and even more preferably 0.20 or more from the viewpoints of improving the feel at the time of application to the hair and suppressing the spread of the hair under high humidity conditions, and is preferably 5.0 or less, more preferably 3.0 or less, still more preferably 2.0 or less, and even more preferably 1.5 or less from the viewpoint of improving the emulsified state. Thus, the mass ratio of the total content of the component (A) and the component (B) to the content of the component (C) in the hair cosmetic composition, [{(A) + (B)}/(C)], is preferably 0.01 or more and 5.0 or less, more preferably 0.05 or more and 3.0 or less, still more preferably 0.10 or more and 2.0 or less, and even more preferably 0.20 or more and 1.5 or less.

In a case where an organic acid is used, the content of the organic acid in the hair cosmetic composition is preferably 0.01% by mass or more, more preferably 0.02% by mass or more, still more preferably 0.03% by mass or more, even more preferably 0.05% by mass or more, even more preferably 0.1% by mass or more, even more preferably 0.2% by mass or more, and even more preferably 0.3% by mass or more, from the viewpoint of facilitating the formation of the polyion complex. On the other hand, from the viewpoint of facilitating the formation of the polyion complex and from the viewpoint of improving the stability of the composition, the content is preferably 5.0% by mass or less, more preferably 3.0% by mass or less, still more preferably 2.0% by mass or less, an even more preferably 1.5% by mass or less. Thus, the content of the organic acid in the hair cosmetic composition is preferably 0.01% by mass or more and 5.0% by mass or less, more preferably 0.02% by mass or more and 3.0% by mass or less, still more preferably 0.03% by mass or more and 3.0% by mass or less, even more preferably 0.05% by mass or more and 3.0% by mass or less, even more preferably 0.1% by mass or more and 2.0% by mass or less, even more preferably 0.1% by mass or more and 1.5% by mass or less, even more preferably 0.2% by mass or more and 1.5% by mass or less, and even more preferably 0.3% by mass or more and 1.5% by mass or less.

However, when the hair cosmetic composition contains any of the alkyldimethylamine and a salt thereof (v), the alkoxyalkyldimethylamine and a salt thereof (vi), and the alkylamidoalkyldimethylamine and a salt thereof (vii) as the component (D), the content of the organic acid in the hair cosmetic composition may be an amount more than 5.0% by mass. This is because the organic acid is expected to improve the stability of the hair cosmetic composition and to improve the feel at the time of application to the hair since the organic acid exists as counter anion of the amine.

When the organic acid is blended in a state of an organic acid salt, the content is an amount converted to an organic acid.

The content of water in the hair cosmetic composition is preferably 10% by mass or more, more preferably 20% by mass or more, and still more preferably 30% by mass or more, from the viewpoint of facilitating the formation of the polyion complex and from the viewpoint of improving the emulsified state. On the other hand, from the viewpoint of the stability of the composition and from the viewpoint of the degree of freedom of blending, the content is preferably 99% by mass or less, more preferably 95% by mass or less, and still more preferably 92% by mass or less. Thus, the content of water in the hair cosmetic composition is preferably 10% by mass or more and 99% by mass or less, more preferably 20% by mass or more and 95% by mass or less, and still more preferably 30% by mass or more and 92% by mass or less.

In a case where a cationic surfactant is used as the component (D), the content of the component (D) in the hair cosmetic composition is an amount of preferably 0.1% by mass or more, more preferably 0.2% by mass or more, still more preferably 0.5% by mass or more, and even more preferably 1.0% by mass or more, from the viewpoint of further improving the feel at the time of application to the hair and the feel of the hair after treatment. On the other hand, from the viewpoint of the stability of the composition, the content is preferably 10% by mass or less, more preferably 5.0% by mass or less, and still more preferably 3.5% by mass or less. Thus, the content of the component (D) in the hair cosmetic composition is preferably 0.1% by mass or more and 10% by mass or less, more preferably 0.2% by mass or more and 5.0% by mass or less, still more preferably 0.5% by mass or more and 3.5% by mass or less, and even more preferably 1.0% by mass or more and 3.5% by mass or less.

In a case where a higher alcohol is used as the component (E), the content of the component (E) in the hair cosmetic composition is preferably 0.1% by mass or more, more preferably 0.2% by mass or more, still more preferably 0.5% by mass or more, and even more preferably 1.0% by mass or more, from the viewpoint of further improving the feel at the time of application to the hair and the feel of the hair after treatment. On the other hand, from the viewpoint of the stability of the composition, the content is an amount of preferably 15% by mass or less, more preferably 12% by mass or less, and still more preferably 10% by mass or less. Thus, the content of the component (E) in the hair cosmetic composition is preferably 0.1% by mass or more and 15% by mass or less, more preferably 0.2% by mass or more and 12% by mass or less, still more preferably 0.5% by mass or more and 12% by mass or less, and even more preferably 1.0% by mass or more and 10% by mass or less.

In a case where a non-aromatic polyol is used as the component (F), the content of the component (F) in the hair cosmetic composition is preferably 0.1% by mass or more, more preferably 0.2% by mass or more, still more preferably 0.5% by mass or more, and even more preferably 1.0% by mass or more, from the viewpoint of further improving the feel at the time of application to the hair and the feel of the hair after treatment and from the viewpoint of improving the moisture retention. On the other hand, from the viewpoint of the stability of the composition, the content is preferably 10% by mass or less, more preferably 5.0% by mass or less, and still more preferably 3.0% by mass or less. Thus, the content of the component (F) in the hair cosmetic composition is preferably 0.1% by mass or more and 10% by mass or less, more preferably 0.2% by mass or more and 10% by mass or less, still more preferably 0.5% by mass or more and 5.0% by mass or less, and even more preferably 1.0% by mass or more and 3.0% by mass or less.

In a case where an oil agent is used as the component (G), the content of the component (G) in the hair cosmetic composition is preferably 0.1% by mass or more, more preferably 0.2% by mass or more, still more preferably 0.5% by mass or more, and even more preferably 1.0% by mass or more, from the viewpoint of further improving the feel at the time of application to the hair and the feel of the hair after treatment. On the other hand, from the viewpoint of improving the feeling in use of the composition, the content is preferably 10% by mass or less, more preferably 5.0% by mass or less, and still more preferably 3.0% by mass or less. Thus, the content of the component (G) in the hair cosmetic composition is preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.0% by mass or less.

In addition, the hair cosmetic composition may contain, as optional components, an anionic surfactant, a nonionic surfactant, an amphoteric surfactant, an aromatic alcohol, an antioxidant, an antidandruff agent, a vitamin agent, a bactericide, an anti-inflammatory agent, a preservative, a chelating agent, a moisturizing agent, a pearl agent, a ceramide, a perfume, a plant extract, an ultraviolet absorber, a pH modifier and the like.

### (pH)

The pH of a 5% aqueous solution of the hair cosmetic composition at 25°C is preferably 2.0 or more, and more preferably 3.0 or more, and is preferably 10.0 or less, more preferably 7.0 or less, and still more preferably 5.0 or less, from the viewpoint of improving the feel when the hair cosmetic composition is applied to the hair. Thus, the pH of a 5% aqueous solution of the hair cosmetic composition at 25°C is preferably 2.0 or more and 10.0 or less, more preferably 3.0 or more and 7.0 or less, and still more preferably 3.0 or more and 5.0 or less.

The pH can be measured by adding water to the hair cosmetic composition to prepare a 5% aqueous solution and using a pH meter at 25°C.

### (Method for Producing Hair Cosmetic Composition)

The method for producing the hair cosmetic composition is not particularly limited, but in a case where the hair cosmetic composition is an emulsion composition, from the viewpoint that it is possible to produce a highly stable hair cosmetic composition that has a good feel at the time of application to the hair and is capable of suppressing the spread of the hair under high humidity conditions, it is preferable to produce the hair cosmetic composition by a method having the following step (1) and any of the following step (2-1) or step (2-2). step (1): a step for preparing a mixture A containing component (A), component (B), an organic acid, and water;
step (2-1): a step for preparing an emulsion (I) by mixing the mixture A with an oil phase component containing an emulsion forming component;
step (2-2): a step for preparing an emulsion (II) by mixing an oil phase component containing an emulsion forming component with an aqueous phase component other than the mixture A, followed by mixing with the mixture A.

In the description herein, the "emulsion forming component" means an oily component for forming an emulsion contained in the emulsion composition.

In the description herein, the "aqueous phase component" means a component constituting the aqueous phase in the production step of the emulsion composition, and the "oil phase component" means a component constituting the oil phase in the production step of the emulsion composition.

The component (C) may be mixed in any step other than the step (1). The component (C) is preferably mixed after the step (2-1) or after the step (2-2) from the viewpoint of preparing a highly stable emulsion composition. More preferably, the emulsion (I) and the component (C) are mixed after preparing the emulsion (I) in the step (2-1), or the component (C) is mixed after mixing the emulsion (II) and the mixture A in the step (2-2).

### <Step (1): Step of Preparing Mixture A>

In the step (1), the component (A), the component (B), the organic acid, and water are mixed to prepare a mixture A (aqueous phase component) containing the component (A), the component (B), the organic acid, and water. By performing the step (1), a mixture A containing a polyion complex formed from the component (A) and the component (B) is obtained.

In the preparation of the mixture A, the mixing order of the component (A), the component (B), the organic acid, and water is not particularly limited, and, for example, the component (A), the component (B), and the organic acid may be added to water at the same time and mixed. From the viewpoint of facilitating the formation of the polyion complex and from the viewpoint of forming a more homogeneous polyion complex in water, it is preferable that the component (A) and the organic acid are first added to water and mixed, and then the component (B) is added and mixed.

The temperature at the time of mixing in the step (1) is not particularly limited, but is preferably 40°C or higher, more preferably 45°C or higher, and still more preferably 50°C or higher, from the viewpoint of improving the dispersibility of the component (A) and the component (B) and from the viewpoint of facilitating the formation of the polyion complex, and is preferably 90°C or lower, more preferably 80°C or lower, further preferably 70°C or lower, and further preferably 65°C or lower, from the viewpoint of suppressing thermal deterioration of the blending components. Thus, the temperature at the time of mixing in the step (1) is preferably 40°C or higher and 90°C or lower, more preferably 45°C or higher and 80°C or lower, still more preferably 50°C or higher and 70°C or lower, and even more preferably 50°C or higher and 65°C or lower.

The mixing time in the step (1) is not particularly limited, but is preferably 1 minute or more, and more preferably 3 minutes or more, from the viewpoint of improving the emulsified state, and is preferably 1 hour or less from the viewpoint of productivity.

The mixing in the step (1) can be performed using a known emulsifying machine or stirring device such as a high-pressure emulsifying machine, an ultrasonic emulsifying machine, and a homomixer.

The mixture A obtained in the step (1) may be a transparent solution, but is preferably in a state of a suspension. In the mixture A in a suspension state, it is considered that the polyion complex formed from the component (A) and the component (B) is not solubilized but is precipitated in the solution and uniformly dispersed. Then, it is considered that when the mixture A exhibiting a suspension state is used in the step (2-1) or (2-2), the state of the stable polyion complex is maintained in the obtained emulsion composition, and the stability of the emulsion composition, the feel at the time of application to the hair, and the effect of suppressing the spread of the hair under high humidity conditions are further improved.

The blending amount of the component (A) in the mixture A is an amount of preferably 0.01% by mass or more, more preferably 0.03% by mass or more, and still more preferably 0.05% by mass or more, based on 100% by mass of the total amount of the mixture A, from the viewpoint of facilitating the formation of the polyion complex, from the viewpoint of improving the feel at the time of application of the obtained composition to the hair, and from the viewpoint of suppressing the spread of the hair under high humidity conditions. On the other hand, from the viewpoint of improving the emulsified state, the blending amount is preferably less than 10% by mass, more preferably less than 5% by mass, still more preferably less than 1% by mass, and even more preferably less than 0.5% by mass. Thus, the blending amount of the component (A) in the mixture A is preferably 0.01% by mass or more and less than 10% by mass, more preferably 0.03% by mass or more and less than 5% by mass, still more preferably 0.03% by mass or more and less than 1% by mass, and even more preferably 0.05% by mass or more and less than 0.5% by mass based on 100% by mass of the total amount of the mixture A.

The blending amount of the component (B) in the mixture A is an amount of preferably 0.01% by mass or more, more preferably 0.02% by mass or more, and still more preferably 0.05% by mass or more, based on 100% by mass of the total amount of the mixture A, from the viewpoint of facilitating the formation of the polyion complex, from the viewpoint of improving the feel at the time of application of the obtained composition to the hair, and from the viewpoint of suppressing the spread of the hair under high humidity conditions. On the other hand, from the viewpoint of improving the emulsified state, the blending amount is preferably less than 5% by mass, more preferably less than 3% by mass, still more preferably less than 1% by mass, and even more preferably less than 0.3% by mass. Thus, the blending amount of the component (B) in the mixture A is preferably 0.01% by mass or more and less than 5% by mass, more preferably 0.01% by mass or more and less than 3% by mass, still more preferably 0.02% by mass or more and less than 1% by mass, and even more preferably 0.05% by mass or more and less than 0.3% by mass based on 100% by mass of the total amount of the mixture A.

The blending amount of the organic acid in the mixture A is an amount of preferably 0.01% by mass or more, more preferably 0.03% by mass or more, and still more preferably 0.05% by mass or more, based on 100% by mass of the total amount of the mixture A, from the viewpoint of facilitating the formation of the polyion complex. On the other hand, from the viewpoint of facilitating the preparation of the mixture A in a suspension state, the blending amount is preferably less than 10% by mass, more preferably less than 5% by mass, still more preferably less than 1% by mass, even more preferably less than 0.75% by mass, and even more preferably less than 0.30% by mass. Thus, the blending amount of the organic acid in the mixture A is preferably 0.01% by mass or more and less than 10% by mass, more preferably 0.01% by mass or more and less than 5% by mass, still more preferably 0.03% by mass or more and less than 1% by mass, even more preferably 0.05% by mass or more and less than 0.75% by mass, and even more preferably 0.05% by mass or more and less than 0.30% by mass based on 100% by mass of the total amount of the mixture A.

The amount of the organic acid used for the preparation of the mixture A in the step (1) may be a part of the organic acid contained in the obtained composition.

In the mixture A, the mass ratio of the total blending amount of the component (A) and the component (B) to the blending amount of the organic acid [(A + B)/organic acid] is preferably in a range of 1/0.1 to 1/20, more preferably 1/0.1 to 1/15, still more preferably 1/0.20 to 1/10, even more preferably 1/0.20 to 1/8, and even more preferably 1/0.22 to 1/4, from the viewpoint of facilitating the preparation of the mixture A in a suspension state, from the viewpoint of improving the feel at the time of application to the hair, and from the viewpoint of suppressing the spread of the hair under high humidity conditions.

The blending amount of water in the mixture A is an amount of preferably 50% by mass or more, more preferably 60% by mass or more, still more preferably 70% by mass or more, and even more preferably 80% by mass or more, and is an amount of preferably 99.97% by mass or less and preferably 99.8% by mass or less, based on 100% by mass of the total amount of the mixture A, from the viewpoint of facilitating the formation of the polyion complex and from the viewpoint of improving the emulsified state. Thus, the blending amount of water in the mixture A is an amount of preferably 50% by mass or more and 99.97% by mass or less, more preferably 50% by mass or more and 99.8% by mass or less, still more preferably 60% by mass or more and 99.8% by mass or less, even more preferably 70% by mass or more and 99.8% by mass or less, and even more preferably 80% by mass or more and 99.8% by mass or less, based on 100% by mass of the total amount of the mixture A.

The amount of water used for the preparation of the mixture A in the step (1) may be a part of water contained in the obtained composition.

The mixture A may contain components other than the component (A), the component (B), the organic acid, and water, but from the viewpoint of facilitating the formation of the polyion complex, from the viewpoint of improving the emulsified state, and from the viewpoint of stability of the composition, the total content of the component (A), the component (B), the organic acid, and water in the mixture A is preferably 70% by mass or more, more preferably 80% by mass or more, still more preferably 90% by mass or more, and even more preferably 95% by mass or more, and is 100% by mass or less.

In the step (1), from the viewpoint of more uniformly dispersing the polyion complex in the mixture A, the pH of the mixture A at 25°C is preferably 1.5 or more, and more preferably 2.0 or more, and is preferably 4.0 or less, more preferably 3.2 or less, and still more preferably 3.0 or less. Thus, the pH of the mixture A at 25°C is preferably 1.5 or more and 4.0 or less, more preferably 2.0 or more and 4.0 or less, still more preferably 2.0 or more and 3.2 or less, and even more preferably 2.0 or more and 3.0 or less.

The pH of the mixture A at 25°C can be measured using a pH meter.

### <Step (2)>

When the hair cosmetic composition is an emulsion composition, the method for producing the hair cosmetic composition preferably includes, after the step (1), any of the following step (2-1) or step (2-2). This makes it possible to produce an emulsion composition having high stability and containing the polyion complex obtained in the step (1).
Step (2-1): a step for preparing an emulsion (I) by mixing the mixture A with an oil phase component containing an emulsion forming component;
Step (2-2): a step for preparing an emulsion (II) by mixing an oil phase component containing an emulsion forming component with an aqueous phase component other than the mixture A, followed by mixing with the mixture A.

### (Step (2-1))

In the step (2-1), first, the mixture A obtained in the step (1) and an oil phase component containing an emulsion forming component are mixed to prepare an emulsion (I). Then, an oil phase component containing the component (C) is preferably mixed. Further, the aqueous phase component other than the mixture A can be mixed as required.

The mixing ratio of the mixture A and the oil phase component containing an emulsion forming component may be adjusted and mixed so that the total blending amount of the component (A) and the component (B) in the obtained emulsion composition is within a predetermined range, and is not particularly limited.

The "oil phase component containing an emulsion forming component" preferably contains, as emulsion forming components, component (D): a cationic surfactant, and component (E): a higher alcohol, and may further contain component (F): a non-aromatic polyol.

In the oil phase component containing the emulsion forming component used in the step (2-1), the content of the emulsion forming component is preferably 30% by mass or more, more preferably 50% by mass or more, and still more preferably 70% by mass or more, and is 100% by mass or less from the viewpoint of stably forming an emulsion.

The "oil phase component containing the component (C)" is an oil phase component containing the component (C) and not containing an emulsion forming component, and may further contain component (G): an oil agent in addition to the component (C).

Examples of the "aqueous phase component other than the mixture A" include the organic acid and the remaining amount of water, and the component (F) (non-aromatic polyol).

The temperature at the time of mixing the mixture A and the oil phase component containing the emulsion forming component in the step (2-1) is not particularly limited, but is preferably 40°C or higher, more preferably 45°C or higher, and still more preferably 50°C or higher from the viewpoint of the stability of the obtained composition, and from the viewpoint of suppressing thermal deterioration of the blending components, is preferably 90°C or lower, and more preferably 80°C or lower. Thus, the temperature at the time of mixing the mixture A and the oil phase component containing the emulsion forming component in the step (2-1) is preferably 40°C or higher and 90°C or lower, more preferably 45°C or higher and 90°C or lower, and still more preferably 50°C or higher and 80°C or lower.

The mixing time of the mixture A and the oil phase component containing the emulsion forming component in the step (2-1) is not particularly limited, but is preferably 1 minute or more, and more preferably 3 minutes or more, from the viewpoint of improving the emulsified state, and is preferably 1 hour or less from the viewpoint of productivity.

Next, the obtained emulsion (I) is mixed with the oil phase component containing the above-mentioned component (C) and, if necessary, the aqueous phase component (other components) other than the mixture A.

The emulsion (I) and the oil phase component containing the component (C) and the aqueous phase component other than the mixture A may be mixed in one batch or may be divided into several batches, with one added to the other.

The temperature at the time of mixing the emulsion (I) with the oil phase component containing the component (C) and the aqueous phase component other than the mixture A is not particularly limited, but is preferably 40°C or higher, more preferably 45°C or higher, and still more preferably 50°C or higher, from the viewpoint of improving the feel at the time of application of the obtained composition to the hair, and from the viewpoint of improving the stability of the emulsion composition, and is preferably 90°C or lower, more preferably 80°C or lower, and still more preferably 70°C or lower, from the viewpoint of suppressing thermal deterioration of the blending components. When heating is performed during the preparation of the emulsion (I), it is preferable to mix the emulsion (I) with other components while performing a cooling step. The cooling step can be performed by a known method such as air cooling.

The mixing time of the emulsion (I) with other components is also not particularly limited, and is preferably 5 minutes or more, and more preferably 10 minutes or more, and from the viewpoint of productivity, is preferably 12 hours or less, more preferably 5 hours or less, and still more preferably 1 hour or less.

### (Step (2-2))

In the step (2-2), first, an oil phase component containing an emulsion forming component and an aqueous phase component other than the mixture A are mixed to prepare an emulsion (II). Next, the emulsion (II) and the mixture A are mixed.

The "oil phase component containing an emulsion forming component" and the "aqueous phase component other than the mixture A" are the same as in the step (2-1).

The aqueous phase component other than the mixture A used for the preparation of the emulsion (II) does not need to be the total amount of the aqueous phase components used in the step (2-2), and may be at least a part thereof. The aqueous phase component other than the mixture A used for the preparation of the emulsion (II) is preferably water.

The temperature at the time of mixing the oil phase component containing the emulsion forming component and the aqueous phase component other than the mixture A in the step (2-2) is not particularly limited, but is preferably 40°C or higher, more preferably 45°C or higher, and still more preferably 50°C or higher, from the viewpoint of improving the feel at the time of application of the obtained composition to the hair, and from the viewpoint of improving the stability of the emulsion composition, and is preferably 90°C or lower, and more preferably 80°C or lower, from the viewpoint of suppressing thermal deterioration of the blending components. Thus, the temperature at the time of mixing of the oil phase component containing the emulsion forming component and the aqueous phase component other than the mixture A in the step (2-2) is preferably 40°C or higher and 90°C or lower, more preferably 45°C or higher and 90°C or lower, and still more preferably 50°C or higher and 80°C or lower. The mixing time is also not particularly limited, but is preferably 1 minute or more, and more preferably 3 minutes or more, from the viewpoint of preparing an emulsion, and is preferably 1 hour or less, from the viewpoint of productivity.

Next, the obtained emulsion (II) and the mixture A are mixed.

The mixing ratio of the mixture A and the emulsion (II) may be adjusted and mixed so that the total blending amount of the component (A) and the component (B) in the obtained emulsion composition is within a predetermined range, and is not particularly limited.

The emulsion (II) and the mixture A may be mixed in one batch or may be divided into several batches, with one added to the other. In addition, at the same time as the mixing of the emulsion (II) and the mixture A, or before or after the mixing (preferably after the mixing of the emulsion (II) and the mixture A), an oil phase component containing the component (C) is mixed. Further, if necessary, the remaining amount of the aqueous phase component can be added and mixed.

The temperature at the time of mixing of the emulsion (II) and the mixture A is not particularly limited, but is preferably 50°C or lower, and more preferably 45°C or lower, and is preferably 10°C or higher, and more preferably 20°C or higher, from the viewpoint of stability of the obtained emulsion composition. Thus, the temperature at the time of mixing of the emulsion (II) and the mixture A is preferably 10°C or higher and 50°C or lower, and more preferably 20°C or higher and 45°C or lower.

The mixing time of the emulsion (II) with the mixture A is not particularly limited, and is preferably 1 minute or more, and more preferably 3 minutes or more, and from the viewpoint of productivity, the mixing time is preferably 1 hour or less.

Each mixing step in the step (2-1) and the step (2-2) can be performed using the same apparatus as that in the step (1).

In addition, from the viewpoint of making the emulsified state more favorable, the method for producing a hair cosmetic composition preferably includes the step (1) and the step (2-1).

With respect to the above-described embodiments, the present invention further discloses the following.
<1> A hair cosmetic composition containing:
   component (A): one or more polymers selected from the group consisting of a cationic polymer and an amphoteric polymer;
   component (B): one or more anionic polymers selected from the group consisting of a polyacrylic acid, a (meth)acrylic acid/alkyl (meth)acrylate copolymer, and a salt thereof; and
   component (C): one or more modified silicones selected from the group consisting of an amino-modified silicone and an aminopolyether-modified silicone,
   in which the component (B) has a weight average molecular weight of 3,000 or more and 50,000 or less, a total content of the component (A) and the component (B) is less than 1.0% by mass, and a content of the component (C) is 0.03% by mass or more.
<2> A hair cosmetic composition containing:
   component (A): one or more polymers selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), and an acrylic acid-methyl acrylate-methacrylamidopropyltrimethylammonium chloride copolymer (polyquaternium-47);
   component (B): one or more anionic polymers selected from the group consisting of a polyacrylic acid, an (acrylic acid/stearyl acrylate) copolymer, or a salt thereof; and
   component (C): one or more modified silicones selected from the group consisting of an amino-modified silicone and an aminopolyether-modified silicone,
   in which the component (B) has a weight average molecular weight of 3,000 or more and 50,000 or less, a total content of the component (A) and the component (B) is 0.05% by mass or more and 0.3% by mass or less, and a content of the component (C) is 0.1% by mass or more and 1.0% by mass or less.
<3> The hair cosmetic composition according to <1>, in which the cationic polymer is a polymer having a cationic group and substantially having no anionic group and amphoteric group.
<4> The hair cosmetic composition according to <1> or <3>, in which the cationic polymer has a cationic group, and a molar amount of the anionic group and the amphoteric group with respect to the cationic group is preferably 0.1% or less.
<5> The hair cosmetic composition according to any one of <1>, <3>, and <4>, in which the amphoteric polymer is preferably a polymer having a cationic group and an anionic group, a pH of a 1% aqueous solution of the polymer at 25°C is less than 5.1, and the polymer is positively charged as the total charge of the polymer.
<6> The hair cosmetic composition according to any one of <1> to <5>, in which a cationic charge density of the component (A) is preferably 0.1 meq/g or more and 10 meq/g or less, more preferably 0.5 meq/g or more and 8.0 meq/g or less, still more preferably 1.0 meq/g or more and 7.0 meq/g or less, and even more preferably 2.0 meq/g or more and 7.0 meq/g or less.
<7> The hair cosmetic composition according to any one of <1> to <6>, in which a weight average molecular weight of the component (A) is preferably 5,000 or more and 2,000,000 or less and more preferably 8,000 or more and 1,500,000 or less.
<8> The hair cosmetic composition according to any one of <1>, and <3> to <7>, in which the component (A) is preferably one or more selected from the group consisting of a cationized guar gum, a cationized tara gum, a cationized locust bean gum, a cationic starch, a cationized cellulose, a cationized hydroxyalkyl cellulose, a cationized polyvinyl alcohol, a polyethyleneimine, a quaternized dialkylaminoalkyl (meth)acrylate polymer, a diallyl quaternized ammonium salt polymer, a methacrylamidopropyltrimethylammonium salt polymer, a methacryloyl ethyltrimethylammonium salt polymer, a vinyl imidazolium trichloride-vinyl pyrrolidone copolymer (polyquaternium-16), a vinyl pyrrolidone-alkylamino (meth)acrylate copolymer, a vinyl pyrrolidone-alkylamino (meth)acrylate-vinyl caprolactam copolymer, an alkyl acrylamide-(meth)acrylate-alkylamino alkylacrylamide-polyethylene glycol (meth)acrylate copolymer, and an adipic acid-dimethylaminohydroxypropyl ethylenetriamine copolymer, more preferably one or more selected from the group consisting of a quaternized dialkylaminoalkyl (meth)acrylate polymer, a diallyl quaternized ammonium salt polymer, a methacrylamidopropyl trimethylammonium salt polymer, a methacryloyl ethyl trimethylammonium salt polymer, and a vinyl imidazolium trichloride-vinyl pyrrolidone copolymer (polyquaternium-16), still more preferably one or more selected from the group consisting of a diallyl quaternized ammonium salt polymer, a methacrylamidopropyl trimethylammonium salt polymer, and a vinyl imidazolium trichloride-vinyl pyrrolidone copolymer (polyquaternium-16), and even more preferably one or more selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), an acrylic acid-methyl acrylate-methacrylamidopropyl trimethylammonium chloride copolymer (polyquaternium-47), and a vinyl imidazolium trichloride-vinyl pyrrolidone copolymer (polyquaternium-16).
<9> The hair cosmetic composition according to any one of <1> to <8>, in which the component (A) is even more preferably one or more selected from the group consisting of a dimethyldiallylammonium chloride polymer (polyquaternium-6), an acrylamide-acrylic acid-dimethyldiallylammonium chloride copolymer (polyquaternium-39), and a vinylimidazolium trichloride-vinylpyrrolidone copolymer (polyquaternium-16).
<10> The hair cosmetic composition according to any one of <2>, <8>, and <9>, in which the cationic charge density of the dimethyldiallylammonium chloride polymer (polyquaternium-6) is even more preferably 2.0 meq/g or more, even more preferably 2.5 meq/g or more, and is even more preferably 6.5 meq/g or less, and the weight average molecular weight is still more preferably 10,000 or more and 200,000 or less.
<11> The hair cosmetic composition according to <2> or <8>, in which the cationic charge density of the dimethyldiallylammonium chloride-acrylic acid copolymer (polyquaternium-22) is even more preferably 2.0 meq/g or more, even more preferably 3.0 meq/g or more, and even more preferably 4.0 meq/g or more, and is even more preferably 6.5 meq/g or less and even more preferably 6.0 meq/g or less, and the weight average molecular weight is still more preferably 20,000 or more, even more preferably 50,000 or more, even more preferably 100,000 or more, even more preferably 200,000 or more, and even more preferably 300,000 or more, and is still more preferably 1,000,000 or less, even more preferably 600,000 or less, and even more preferably 500,000 or less.
<12> The hair cosmetic composition according to any one of <2>, <8>, and <9>, in which the cationic charge density of the acrylamide-acrylic acid-dimethyldiallylammonium chloride copolymer (polyquaternium-39) is even more preferably 2.0 meq/g or more, and even more preferably 2.5 meq/g or more, and is even more preferably 6.5 meq/g or less, even more preferably 5.0 meq/g or less, and even more preferably 4.0 meq/g or less, and the weight average molecular weight is still more preferably 20,000 or more, even more preferably 50,000 or more, and even more preferably 100,000 or more, and is still more preferably 1,000,000 or less, even more preferably 600,000 or less, even more preferably 500,000 or less, and even more preferably 300,000 or less.
<13> The hair cosmetic composition according to <2> or <8>, in which the cationic charge density of the acrylic acid-methyl acrylate-methacrylamidopropyl trimethylammonium chloride copolymer (polyquaternium-47) is even more preferably 2.0 meq/g or more, and even more preferably 2.5 meq/g or more, and is even more preferably 6.5 meq/g or less, even more preferably 5.0 meq/g or less, and even more preferably 4.5 meq/g or less, and the weight average molecular weight is still more preferably 200,000 or more, even more preferably 500,000 or more, and even more preferably 1,000,000 or more, and is preferably 5,000,000 or less, more preferably 3,000,000 or less, and still more preferably 1,500,000 or less.
<14> The hair cosmetic composition according to any one of <2>, <8>, and <9>, in which the cationic charge density of the vinyl imidazolium trichloride-vinyl pyrrolidone copolymer (polyquaternium-16) is even more preferably 2.0 meq/g or more, and is even more preferably 6.5 meq/g or less, even more preferably 5.0 meq/g or less, and even more preferably 4.5 meq/g or less, and the weight average molecular weight is still more preferably 20,000 or more, and even more preferably 50,000 or more, and is still more preferably 150,000 or less.
<15> The hair cosmetic composition according to any one of <1> to <14>, in which an anionic charge density of the component (B) is preferably 0.1 meq/g or more and 30 meq/g or less, more preferably 0.5 meq/g or more and 30 meq/g or less, still more preferably 1.0 meq/g or more and 20 meq/g or less, and even more preferably 2.0 meq/g or more and 15 meq/g or less.
<16> The hair cosmetic composition according to any one of <1> to <15>, in which a weight average molecular weight of the component (B) is preferably 5,000 or more and 40,000 or less and more preferably 10,000 or more and 30,000 or less.
<17> The hair cosmetic composition according to any one of <1>, and <3> to <16>,in which the component (B) is preferably one or more selected from the group consisting of a polyacrylic acid, a (meth)acrylic acid/alkyl (meth)acrylate copolymer, or a salt thereof, and even more preferably one or more selected from the group consisting of a polyacrylic acid, an (acrylic acid/stearyl acrylate) copolymer, or a salt thereof.
<18> The hair cosmetic composition according to any one of <1> to <17>, in which the amino-modified silicone is an amino-modified silicone represented by the following general formula (I): 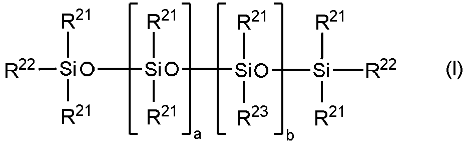 in which each R²¹ independently represents a methyl group or a hydroxy group; each R²² independently represents an alkyl group having 1 or more and 30 or less carbon atoms, a hydroxy group, or R²³; R²³ represents a monovalent group represented by -R²⁴-Z¹ (R²⁴ represents a single bond or a divalent hydrocarbon group having 1 or more and 20 or less carbon atoms, and Z¹ represents a primary to tertiary amino group-containing group or an ammonium group-containing group); a represents a number of 0 or more and 3,000 or less; and b represents a number of 1 or more and 3,000 or less.
<19> The hair cosmetic composition according to <18>, in which the amino-modified silicone represented by the general formula (I) is one or more selected from the group consisting of amodimethicone, aminopropyl dimethicone, bis(aminopropyl)dimethicone, and bis(cetearyl)amodimethicone, preferably an amino-modified silicone represented by any one of the following general formulae (Ia) to (Id), more preferably an amino-modified silicone represented by the following general formula (Ia) (amodimethicone): 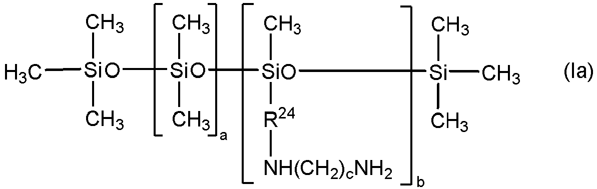 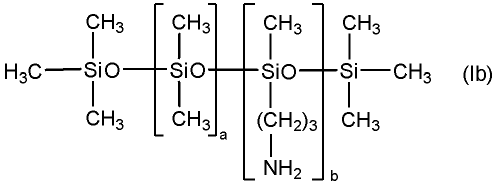 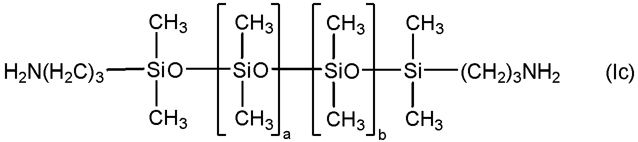 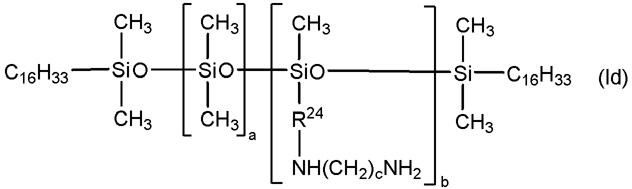 in which, a, b, c, and R²⁴ are the same as described above.
<20> The hair cosmetic composition according to any one of <1> to <19>, in which the amino-modified silicone has a kinematic viscosity at 25°C of preferably 20 mm²/s or more and 10,000 mm²/s or less, more preferably 100 mm²/s or more and 5,000 mm²/s or less, and still more preferably 500 mm²/s or more and 2,000 mm²/s or less.
<21> The hair cosmetic composition according to any one of <1> to <20>, in which when the number of moles of silicon atoms in the aminopolyether-modified silicone is Si and the average addition mole number of alkylene oxide is AO, Si/AO is preferably 0.05 or more and 3.0 or less, more preferably 0.1 or more and 2.8 or less, still more preferably 0.3 or more and 2.8 or less, even more preferably 0.5 or more and 2.6 or less, and even more preferably 0.7 or more and 2.4 or less.
<22> The hair cosmetic composition according to any one of <1> to <21>, in which a nitrogen content of the aminopolyether-modified silicone is preferably 0.1% by mass or more and 2.5% by mass or less, more preferably 0.2% by mass or more and 2.0% by mass or less, still more preferably 0.5% by mass or more and 1.8% by mass or less, and even more preferably 1.0% by mass or more and 1.5% by mass or less.
<23> The hair cosmetic composition according to any one of <1> to <22>, in which the aminopolyether-modified silicone is preferably an aminopolyether-modified silicone represented by the following general formula (II): 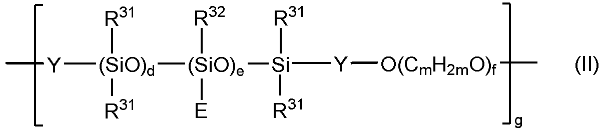 in which, R³¹ represents a hydrogen atom or a monovalent hydrocarbon group having 1 or more and 6 or less carbon atoms; R³² represents any one of R³¹ and E; E represents a monovalent group represented by -R³³-Z² (R³³ represents a single bond or a divalent hydrocarbon group having 1 or more and 20 or less carbon atoms; Z² represents a primary to tertiary amino group-containing group or an ammonium group-containing group); Y represents an alkylene group having 1 or more and 6 or less carbon atoms; d represents a number of 2 or more; e represents a number of 1 or more; f represents a number of 2 or more and 100 or less; g represents a number of 1 or more; and m represents a number of 2 or more and 10 or less; the bonding order between the structural units in parentheses is not limited, and the bonding form may be a block shape or a random shape; f number of CₘH₂ₘO may be the same or different; and a plurality of R³¹, R³², E, and Y may be the same or different.
<24> The hair cosmetic composition according to <23>, in which the aminopolyether-modified silicone is more preferably an aminopolyether-modified silicone represented by the following general formula (IIa): 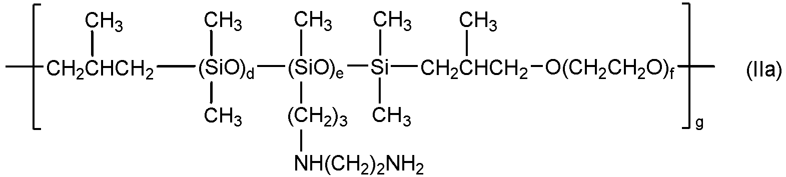 in which, d to g are the same as described above.
<25> The hair cosmetic composition according to any one of <1> to <24>, in which the aminopolyether-modified silicone has a kinematic viscosity at 25°C of preferably 5,000 mm²/s or more and 20,000 mm²/s or less, more preferably 20,000 mm²/s or more and 150,000 mm²/s or less, and still more preferably 40,000 mm²/s or more and 100,000 mm²/s or less.
<26> The hair cosmetic composition according to any one of <1> to <25>, in which a content of the component (A) in the hair cosmetic composition is preferably 0.01% by mass or more and less than 0.50% by mass, more preferably 0.02% by mass or more and 0.30% by mass or less, and still more preferably 0.03% by mass or more and 0.20% by mass or less.
<27> The hair cosmetic composition according to any one of <1> to <26>, in which a content of the component (B) in the hair cosmetic composition is preferably 0.01% by mass or more and less than 0.50% by mass, more preferably 0.01% by mass or more and 0.30% by mass or less, still more preferably 0.01% by mass or more and 0.20% by mass or less, and even more preferably 0.015% by mass or more and 0.10% by mass or less.
<28> The hair cosmetic composition according to any one of <1>, and <3> to <27>, in which a total content of the component (A) and the component (B) in the hair cosmetic composition is preferably 0.01% by mass or more and 0.9% by mass or less, more preferably 0.03% by mass or more and 0.9% by mass or less, still more preferably 0.05% by mass or more and 0.9% by mass or less, even more preferably 0.05% by mass or more and 0.5% by mass or less, and even more preferably 0.05% by mass or more and 0.3% by mass or less.
<29> The hair cosmetic composition according to any one of <1> to <28>, in which a ratio of number of moles of cationic charges of the component (A) to number of moles of anionic charges of the component (B) in the hair cosmetic composition is in an amount of preferably 5/95 to 95/5, more preferably 10/90 to 90/10, still more preferably 20/80 to 80/20, even more preferably 20/80 to 60/40, and even more preferably 40/60 to 60/40.
<30> The hair cosmetic composition according to any one of <1>, and <3> to <29>, in which a content of the component (C) in the hair cosmetic composition is preferably 0.03% by mass or more and 5.0% by mass or less, more preferably 0.05% by mass or more and 5.0% by mass or less, still more preferably 0.05% by mass or more and 2.0% by mass or less, even more preferably 0.1% by mass or more and 1.0% by mass or less, and even more preferably 0.1% by mass or more and 0.5% by mass or less.
<31> The hair cosmetic composition according to any one of <1> to <30>, in which the mass ratio of the total content of the component (A) and the component (B) to the content of the component (C) in the hair cosmetic composition, [{ (A) + (B)}/(C)], is preferably 0.01 or more and 5.0 or less, more preferably 0.05 or more and 3.0 or less, still more preferably 0.10 or more and 2.0 or less, and even more preferably 0.20 or more and 1.5 or less.
<32> The hair cosmetic composition according to any one of <1> to <31>, further containing an organic acid.
<33> The hair cosmetic composition according to <32>, in which the organic acid is one or more selected from the group consisting of carboxylic acid-based compounds and sulfonic acid-based compounds other than the component (B).
<34> The hair cosmetic composition according to <32> or <33>, in which the organic acid is a compound having a molecular weight of preferably 500 or less and more preferably 200 or less.
<35> The hair cosmetic composition according to any one of <32> to <34>, in which the organic acid is preferably one or more selected from the group consisting of an aliphatic dicarboxylic acid, hydroxycarboxylic acid, and an aromatic sulfonic acid, more preferably one or more selected from the group consisting of succinic acid, lactic acid, malic acid, glycolic acid, p-toluenesulfonic acid, and naphthalenesulfonic acid, still more preferably one or more selected from the group consisting of succinic acid and lactic acid, and even more preferably lactic acid.
<36> The hair cosmetic composition according to any one of <32> to <35>, in which a content of the organic acid in the hair cosmetic composition is preferably 0.01% by mass or more and 5.0% by mass or less, more preferably 0.02% by mass or more and 3.0% by mass or less, still more preferably 0.03% by mass or more and 3.0% by mass or less, even more preferably 0.05% by mass or more and 3.0% by mass or less, even more preferably 0.1% by mass or more and 2.0% by mass or less, even more preferably 0.1% by mass or more and 1.5% by mass or less, even more preferably 0.2% by mass or more and 1.5% by mass or less, and even more preferably 0.3% by mass or more and 1.5% by mass or less.
<37> The hair cosmetic composition according to any one of <1> to <36>, further containing water.
<38> The hair cosmetic composition according to <37>, in which a content of water in the hair cosmetic composition is preferably 10% by mass or more and 99% by mass or less, more preferably 20% by mass or more and 95% by mass or less, and still more preferably 30% by mass or more and 92% by mass or less.
<39> The hair cosmetic composition according to any one of <1> to <38>, further containing a cationic surfactant as component (D).
<40> The hair cosmetic composition according to <39>, in which the component (D) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof, more preferably one or more selected from the group consisting of (i) an alkyltrimethylammonium salt, (ii) an alkoxyalkyltrimethylammonium, and (v) an alkyldimethylamine and a salt thereof, still more preferably one or more selected from the group consisting of behenyltrimethylammonium chloride, stearoxypropyltrimethylammonium chloride, stearoxyethyltrimethylammonium chloride, stearoxyhydroxypropyltrimethylammonium chloride, N,N-dimethyl-3-hexadecyloxypropylamine or a lactic acid salt thereof, and N,N-dimethyl-3-octadecyloxypropylamine (stearoxypropyldimethylamine) or a lactic acid salt thereof, and even more preferably one or more selected from the group consisting of N,N-dimethyl-3-octadecyloxypropylamine (stearoxypropyldimethylamine) or a lactic acid salt thereof.
<41> The hair cosmetic composition according to <39> or <40>, in which a content of the component (D) in the hair cosmetic composition is preferably 0.1% by mass or more and 10% by mass or less, more preferably 0.2% by mass or more and 5.0% by mass or less, still more preferably 0.5% by mass or more and 3.5% by mass or less, and even more preferably 1.0% by mass or more and 3.5% by mass or less.
<42> The hair cosmetic composition according to any one of <1> to <41>, further containing a higher alcohol as component (E).
<43> The hair cosmetic composition according to <42>, in which the component (E) is an aliphatic monohydric alcohol having 12 or more carbon atoms, preferably 12 or more and 22 or less carbon atoms, more preferably one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol, still more preferably one or more selected from the group consisting of cetyl alcohol, myristyl alcohol, stearyl alcohol, and behenyl alcohol, and even more preferably stearyl alcohol.
<44> The hair cosmetic composition according to <42> or <43>, in which a content of the component (E) in the hair cosmetic composition is preferably 0.1% by mass or more and 15% by mass or less, more preferably 0.2% by mass or more and 12% by mass or less, still more preferably 0.5% by mass or more and 12% by mass or less, and even more preferably 1.0% by mass or more and 10% by mass or less.
<45> The hair cosmetic composition according to any one of <1> to <44>, further containing a non-aromatic polyol as component (F).
<46> The hair cosmetic composition according to <45>, in which the component (F) is one or more selected from the group consisting of 1,3-butylene glycol, glycerin, ethylene glycol, propylene glycol, and dipropylene glycol, preferably one or more selected from the group consisting of 1,3-butylene glycol and dipropylene glycol, and more preferably dipropylene glycol.
<47> The hair cosmetic composition according to <45> or <46>, in which a content of the component (F) in the hair cosmetic composition is preferably 0.1% by mass or more and 10% by mass or less, more preferably 0.2% by mass or more and 10% by mass or less, still more preferably 0.5% by mass or more and 5.0% by mass or less, and even more preferably 1.0% by mass or more and 3.0% by mass or less.
<48> The hair cosmetic composition according to any one of <1> to <47>, further containing an oil agent other than the component (C) as component (G).
<49> The hair cosmetic composition according to <48>, in which the component (G) is one or more selected from the group consisting of (a) silicone oil, (b) ester oil, (c) ether oil, (d) hydrocarbon oil, (e) higher fatty acid, and (f) wax, preferably one or more selected from the group consisting of (a) silicone oil, (b) ester oil, (d) hydrocarbon oil, and (e) higher fatty acid, and more preferably containing one or more selected from the group consisting of (a) silicone oil and (e) higher fatty acid, still more preferably containing one or more selected from the group consisting of dimethylpolysiloxane, dimethiconol, methylphenylpolysiloxane, modified silicone other than the component (C), and fatty acids having 12 or more carbon atoms, even more preferably containing one or more selected from the group consisting of dimethylpolysiloxane, dimethiconol, polyether-modified silicone, lauric acid, myristic acid, palmitic acid, stearic acid, behenic acid, oleic acid, coconut oil fatty acids, lanolin fatty acids, isostearyl acid, and isopalmitic acid, even more preferably containing one or more selected from the group consisting of dimethylpolysiloxane and lanolin fatty acids, and even more preferably containing dimethylpolysiloxane and lanolin fatty acids.
<50> The hair cosmetic composition according to <48> or <49>, in which a content of the component (G) in the hair cosmetic composition is preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.0% by mass or less.
<51> The hair cosmetic composition according to any one of <1> to <50>, in which the pH at 25°C of a 5% aqueous solution of the hair cosmetic composition is preferably 2.0 or more and 10.0 or less, more preferably 3.0 or more and 7.0 or less, and still more preferably 3.0 or more and 5.0 or less.
<52> A method for producing the hair cosmetic composition according to any one of <1> to, <51>, including the following step (1) and either the following step (2-1) or step (2-2):
   step (1): a step for preparing a mixture A containing component (A), component (B), an organic acid, and water;
   step (2-1): a step for preparing an emulsion (I) by mixing the mixture A with an oil phase component containing an emulsion forming component;
   step (2-2): a step for preparing an emulsion (II) by mixing an oil phase component containing an emulsion forming component with an aqueous phase component other than the mixture A, followed by mixing with the mixture A.
<53> The production method according to <52>, in which the emulsion forming component preferably contains an ionic surfactant and an amphiphilic substance having a hydrophobic group, and more preferably contains component (D): a cationic surfactant and component (E): a higher alcohol.
<54> The production method according to <52> or <53>, in which the component (C) is preferably mixed after the step (2-1) or after the step (2-2), and more preferably, the emulsified product (I) and the component (C) are mixed after the emulsion (I) is prepared in the step (2-1), or the component (C) is mixed after the emulsion (II) and the mixture A are mixed in the step (2-2).
<55> The production method according to any one of <52> to <54>, in which the temperature at the time of mixing in the step (1) is preferably 40°C or higher and 90°C or lower, more preferably 45°C or higher and 80°C or lower, still more preferably 50°C or higher and 70°C or lower, and even more preferably 50°C or higher and 65°C or lower.
<56> The production method according to any one of <52> to <55>, in which the mixing time in the step (1) is preferably 1 minute or more, more preferably 3 minutes or more, and is preferably 1 hour or less.
<57> The production method according to any one of <52> to <56>, in which the mixture A obtained in the step (1) is in a state of a suspension.
<58> The production method according to any one of <52> to <57>, in which a blending amount of the component (A) in the mixture A is an amount of preferably 0.01% by mass or more and less than 10% by mass, more preferably 0.03% by mass or more and less than 5% by mass, still more preferably 0.03% by mass or more and less than 1% by mass, and even more preferably 0.05% by mass or more and less than 0.5% by mass, based on a total amount of 100% by mass of the mixture A.
<59> The production method according to any one of <52> to <58>, in which a blending amount of the component (B) in the mixture A is an amount of preferably 0.01% by mass or more and less than 5% by mass, more preferably 0.01% by mass or more and less than 3% by mass, still more preferably 0.02% by mass or more and less than 1% by mass, and even more preferably 0.05% by mass or more and less than 0.3% by mass, based on a total amount of 100% by mass of the mixture A.
<60> The production method according to any one of <52> to <59>, in which a blending amount of the organic acid in the mixture A is an amount of preferably 0.01% by mass or more and less than 10% by mass, more preferably 0.01% by mass or more and less than 5% by mass, still more preferably 0.03% by mass or more and less than 1% by mass, even more preferably 0.05% by mass or more and less than 0.75% by mass, and even more preferably 0.05% by mass or more and less than 0.30% by mass, based on a total amount of 100% by mass of the mixture A.
<61> The production method according to any one of <52> to <60>, in which in the mixture A, a mass ratio [(A + B)/organic acid] of the total blending amount of the component (A) and the component (B) to the blending amount of the organic acid is in a range of preferably 1/0.1 to 1/20, more preferably 1/0.1 to 1/15, still more preferably 1/0.20 to 1/10, even more preferably 1/0.20 to 1/8, and even more preferably 1/0.22 to 1/4.
<62> The production method according to any one of <52> to <61>, in which a blending amount of water in the mixture A is an amount of preferably 50% by mass or more and 99.97% by mass or less, more preferably 50% by mass or more and 99.8% by mass or less, still more preferably 60% by mass or more and 99.8% by mass or less, even more preferably 70% by mass or more and 99.8% by mass or less, and even more preferably 80% by mass or more and 99.8% by mass or less, based on a total amount of 100% by mass of the mixture A.
<63> The production method according to any one of <52> to <62>, in which a total content of the component (A), the component (B), the organic acid, and water in the mixture A is preferably 70% by mass or more, more preferably 80% by mass or more, still more preferably 90% by mass or more, and even more preferably 95% by mass or more, and is 100% by mass or less.
<64> The production method according to any one of <52> to <63>, in which the pH of the mixture A at 25°C is preferably 1.5 or more and 4.0 or less, more preferably 2.0 or more and 4.0 or less, still more preferably 2.0 or more and 3.2 or less, and even more preferably 2.0 or more and 3.0 or less.
<65> The production method according to any one of <52> to <64>, in which the temperature at the time of mixing of the mixture A and the oil phase component containing the emulsion forming component in the step (2-1) is preferably 40°C or higher and 90°C or lower, more preferably 45°C or higher and 90°C or lower, and still more preferably 50°C or higher and 80°C or lower.
<66> The production method according to any one of <52> to <65>, in which the mixing time of the mixture A and the oil phase component containing the emulsion forming component in the step (2-1) is preferably 1 minute or more, and more preferably 3 minutes or more, and is preferably 1 hour or less.
<67> The production method according to any one of <52> to <66>, in which the temperature at the time of mixing of the oil phase component containing the emulsion forming component and the aqueous phase component other than the mixture A in the step (2-2) is preferably 40°C or higher and 90°C or lower, more preferably 45°C or higher and 90°C or lower, and still more preferably 50°C or higher and 80°C or lower.
<68> The production method according to any one of <52> to <67>, in which the mixing time of the oil phase component containing the emulsion forming component and the aqueous phase component other than the mixture A in the step (2-2) is preferably 1 minute or more, and more preferably 3 minutes or more, and is preferably 1 hour or less.
<69> The production method according to any one of <52> to <68>, in which the temperature at the time of mixing of the emulsion (II) and the mixture A in the step (2-2) is preferably 10°C or higher and 50°C or lower, and more preferably 20°C or higher and 45°C or lower.
<70> The production method according to any one of <52> to <69>, in which the mixing time of the emulsion (II) and the mixture A in the step (2-2) is preferably 1 minute or more, and more preferably 3 minutes or more, and is preferably 1 hour or less.
<71> The production method according to any one of <52> to <70>, in which the method for producing the hair cosmetic composition has the step (1) and the step (2-1).

### Examples

Hereinafter, the present invention will be described with reference to Examples, but the present invention is not limited to the scope of Examples. The measurements and evaluations in Examples were performed by the following methods.

### (pH Measurement)

Water was added to each of the hair cosmetic compositions (hair conditioners) of the examples to prepare a 5% aqueous solution, and the pH at 25°C was measured using a pH meter (F-51, manufactured by HORIBA, Ltd.). The pH was 3.4 in all cases.

### (Measurement of Si/AO in Component (C2))

Ten mg of the component (C2) (aminopolyether-modified silicone) was weighed into a No. 3 screw vial and dried overnight under a nitrogen atmosphere. One mL of heavy chloroform (manufactured by FUJIFILM Wako Pure Chemical Corporation: chloroform-d, 99.8%) was added to and dissolved in the obtained dried product, and the unit ratio of Si/AO was calculated from the integral value of the dimethylsilyl group (6H: -0.3 to 0.3 ppm) bonded to silicon and the integral value of the oxyethylene (EO) chain (4H: 3.3 to 3.9 ppm) by ¹H-NMR (JH074504 AV400, manufactured by BRUKER JAPAN) using the following equation. $\left(Si/AO\right) = \left[\left(Integral value of -0.3 to 0.3 ppm\right)/6\right] / \left[\left(Integral value of 3.3 to 3.9 ppm\right)/4\right]$

### (Emulsified State)

One g of the hair conditioner of each example was dropped on a black plastic underlay, and the appearance and feel when the surface was traced with a finger 10 times were evaluated according to the following criteria.
A: Aggregated particles are not visually recognized, and a smooth feel is felt when the surface is traced with a finger.
B: Aggregated particles are not visually recognized, but a rough feel is felt when the surface is traced with a finger.
C: Aggregated particles are visually recognized, and a rough feel is felt when the surface is traced with a finger.
D: Separated

### (Storage Stability)

The storage stability of the hair conditioner of each example was evaluated by a cycle test.

A cycle test was conducted in which the hair conditioner of each example was stored for two weeks in an environment in which the temperature changes in the range of 40°C to -28°C. The temperature cycle per day was set so that the cycle of the temperature difference ΔT = 40°C was four laps/day. The presence or absence of separation of the hair conditioner after storage was visually observed, and in the tables, the case where there was no separation and it was stable was indicated by "A", and the case where separation was recognized was indicated by "D".

### (Feel at the Time of Application)

A bleaching agent (a mixture of "Kao Foam Hair Bleach L" (first liquid) 7 mL and "Kao Foam Hair Color d" (second liquid) 14 mL) was applied to a tress of Japanese human hair with a length of 30 cm and a mass of 20 g, allowed to stand for 20 minutes, and then sufficiently rinsed with warm water at 35 to 40°C. The above treatment was repeated four times to prepare a tress for evaluation of Japanese damaged hair.

One mL of the hair conditioner of each example was applied to the above-described tress for evaluation, and the hair conditioner was applied to the tress by hand. The feel at that time was evaluated in five grades by five professional panelists according to the following criteria, and the average value was taken as the evaluation result.
5: Very smooth
4: Smooth
3: Slightly smooth
2: Smoothness equivalent to that of the case of using a plain conditioner having the following composition.
1: The presence is immediately lost and the feel is not smooth.

### [Plain Conditioner Composition]

(% by mass)
Stearoxypropyldimethylamine (*1) 0.9
Stearyl alcohol (*2) 3.0
Dipropylene glycol 0.5
Benzyl alcohol 0.2
Lactic acid (*3) 1.1
Purified water Remaining amount
Total 100.0
(*1) "FARMIN DM-E80" manufactured by Kao Corporation, active ingredient amount: 90% by mass
(*2) "KALCOL 8098" manufactured by Kao Corporation, active ingredient amount: 100% by mass
(*3) "PURAC ULTRAPURE 90" manufactured by PURAC Tiland Ltd., active ingredient amount: 90% by mass

### (Moisture Resistance of Hair after Treatment)

One mL of the hair conditioner of each example was applied to the tress for evaluation of Japanese damaged hair produced by the method described above, and the tress was allowed to stand for 30 seconds. After being left for 1 minute in this state, the hair was rinsed with running water for 30 seconds and dried with a dryer until completely dried.

The tress after drying was subjected to 10 times of straight setting while passing through a flat iron ("AHI-938" manufactured by Miki Denki Sangyo Co., Ltd.) set to 160°C using a comb, and a photograph of the tress immediately after setting was taken. Next, the hair after the straight setting was suspended and left to stand for 30 minutes in a high humidity environment of 35°C and 80% RH, and a photograph was taken again. In the two photographs, the width of the tress at a position of 23.5 cm from the root of the tress was measured, and the change in the width of the tress immediately after straight setting and after storage under high humidity was calculated by the following formula. Tress width change value = (tress width after storage under high humidity)/(tress width immediately after straight setting)

When the above value exceeds 1.3, it is visually recognized that the tress is spread, and thus it is determined that the moisture resistance is low.

### Example 1 (Preparation and Evaluation of Hair Conditioner)

A hair conditioner was prepared by the following procedure using the components listed in Table 1.

In a 500 mL beaker, 1.52 g of a polyquaternium-6 solution as the component (A), 3 g of lactic acid (90% by mass), and 422.7 g of water were charged, and heated and stirred at 80°C using a stirring device (mechanical stirrer equipped with two propeller blades). After confirming that the mixture became a uniform solution, 0.37 g of sodium polyacrylate as the component (B) was added and mixed to obtain a mixture A as a uniform suspension (step (1)).

Next, a hair cosmetic composition (hair conditioner) was prepared by the following procedure.

First, 11.0 g of stearoxypropyldimethylamine as the component (D), 31.9 g of stearyl alcohol as the component (E), and 10.0 g of dipropylene glycol described in Table 1 were mixed in advance, and heated and dissolved at 75°C to prepare an oil phase component containing an emulsion forming component. This was added to the mixture A, and heated and stirred at 75°C for 10 minutes. After stirring for 10 minutes, heating was terminated and air cooling was started (preparation of emulsion (I)).

Next, as described in Table 1, 1.0 g of amodimethicone as the component (C), an oil agent (10.0 g of dimethicone and 5.0 g of lanolin fatty acid), and the remaining amount 3.5 g of lactic acid (90% by mass) were added, and the mixture was cooled with stirring until the liquid temperature became 40°C or lower to obtain 500 mL of a hair conditioner (step (2-1)).

The obtained hair conditioner was used to perform evaluation by the above-described methods. The results are shown in Table 1. The blending amounts described in the table of the present examples are the blending amounts (% by mass) of each component as it is, and the "effective content" described in the table of the present examples is the content or blending amount (% by mass) of each component converted into the active ingredient amount.

### Examples 2 to 10 and Comparative Examples 1 to 12

Hair conditioners were produced and evaluated in the same manner as in Example 1, except that the types and amounts of the respective components used in the preparation of the hair conditioner were changed as described in Table 1. The results are shown in Tables 1 and 2.

**Table 1**

| (As it is: % by mass) | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| (A) | Polyquaternium-6 * 1 | | 0.304 | | | | | 0.324 | 0.608 | 0.076 | 0.304 | 0.304 |
| | Polyquaternium-39 *2 | | | 0.363 | | | | | | | | |
| | Polyquaternium-16 *3 | | | | 0.365 | | | | | | | |
| | Polyquaternium-22 *4 | | | | | 0.332 | | | | | | |
| | Polyquaternium-47 *5 | | | | | | 0.731 | | | | | |
| (B) | Sodium polyacrylate *6 | | 0.074 | 0.044 | 0.054 | 0.064 | 0.046 | | 0.148 | 0.018 | 0.074 | 0.074 |
| | (Acrylic acid/stearyl acrylate) copolymer *7 | | | | | | | 0.706 | | | | |
| (B') | Acrylates copolymer *8 | | | | | | | | | | | |
| | (Methyl vinyl ether/maleic acid) copolymer *9 | | | | | | | | | | | |
| (C) | Amodimethicone * 10 | | 0.2 | 0.2 | 0.2 | 0.2 | 0.2 | 0.2 | 0.2 | 0.2 | 2 | |
| | (Bisisobutyl PEG-15/Amodimethicone) copolymer * 11 | | | | | | | | | | | 0.28 |
| (D) | Stearoxypropyldimethylamine * 12 | | 2.2 | 2.2 | 2.2 | 2.2 | 2.2 | 2.2 | 2.2 | 2.2 | 2.2 | 2.2 |
| (E) | Stearyl alcohol * 13 | | 6.38 | 6.38 | 6.38 | 6.38 | 6.38 | 6.38 | 6.38 | 6.38 | 6.38 | 6.38 |
| (F) | Dipropylene glycol *14 | | 2 | 2 | 2 | 2 | 2 | 2 | 2 | 2 | 2 | 2 |
| (G) | Dimethicone *15 | | 2 | 2 | 2 | 2 | 2 | 2 | 2 | 2 | 2 | 2 |
| | Lanolin fatty acid * 16 | | 1 | 1 | 1 | 1 | 1 | 1 | 1 | 1 | 1 | 1 |
| | Lactic acid *17 | | 1.3 | 1.3 | 1.3 | 1.3 | 1.3 | 1.3 | 1.3 | 1.3 | 1.3 | 1.3 |
| | Purified water | | Remaining amount | Remaining amount | Remaining amount | Remaining amount | Remaining amount | Remaining amount | Remaining amount | Remaining amount | Remaining amount | Remaining amount |
| Total | | | 100.00 | 100.00 | 100.00 | 100.00 | 100.00 | 100.00 | 100.00 | 100.00 | 100.00 | 100.00 |
| Component (A) effective content (% by mass) | | | 0.126 | 0.156 | 0.157 | 0.136 | 0.154 | 0.134 | 0.252 | 0.032 | 0.126 | 0.126 |
| Component (B) effective content (% by mass) | | | 0.074 | 0.044 | 0.054 | 0.064 | 0.046 | 0.0706 | 0.148 | 0.018 | 0.074 | 0.074 |
| Component (C) effective content (% by mass) | | | 0.20 | 0.20 | 0.20 | 0.20 | 0.20 | 0.20 | 0.20 | 0.20 | 2.00 | 0.20 |
| Component (D) effective content (% by mass) | | | 1.98 | 1.98 | 1.98 | 1.98 | 1.98 | 1.98 | 1.98 | 1.98 | 1.98 | 1.98 |
| Component (E) effective content (% by mass) | | | 6.60 | 6.60 | 6.60 | 6.60 | 6.60 | 6.60 | 6.60 | 6.60 | 6.60 | 6.60 |
| Component (G) effective content (% by mass) | | | 2.04 | 2.04 | 2.04 | 2.04 | 2.04 | 2.04 | 2.04 | 2.04 | 2.04 | 2.04 |
| Components (A)+(B) effective content (% by mass) | | | 0.20 | 0.20 | 0.21 | 0.20 | 0.20 | 0.21 | 0.40 | 0.05 | 0.20 | 0.20 |
| Mass ratio [(A+B)/C] | | | 1.00 | 1.00 | 1.05 | 1.00 | 1.00 | 1.03 | 2.00 | 0.25 | 0.10 | 1.00 |
| Evaluation results | | Emulsified state | A | A | A | A | A | A | B | A | B | A |
| | | Storage stability | A | A | A | A | A | A | A | A | A | A |
| | | Feel at the time of application | 4.4 | 5 | 4 | 3.8 | 3.8 | 3.6 | 4.2 | 4.6 | 5 | 4.6 |
| | | Moisture resistance of hair after treatment (tress width change value after storage under high humidity condition) | 1.0 | 1.0 | 1.1 | 1.1 | 1.0 | 1.1 | 1.0 | 1.0 | 1.0 | 1.1 |

**Table 2**

| (As it is: % by mass) | | | Comparative Example | | | | | | | | | | | |
|---|---|---|---|---|---|---|---|---|---|---|---|---|---|---|
| | | | 1 | 2 | 3 | 4 | 5 | 6 | 7 | 8 | 9 | 10 | 11 | 12 |
| (A) | Polyquaternium-6 * 1 | | 0.304 | | 0.304 | 1.52 | 0.304 | 0.304 | 0.372 | 0.18 | 0.304 | 0.372 | 0.18 | 2.168 |
| | Polyquaternium-39 *2 | | | | | | | | | | | | | |
| | Polyquaternium-16 *3 | | | | | | | | | | | | | |
| | Polyquaternium-22 *4 | | | | | | | | | | | | | |
| | Polyquaternium-47 *5 | | | | | | | | | | | | | |
| (B) | Sodium polyacrylate *6 | | 0.074 | 0.074 | | 0.369 | 0.074 | | | | | | | |
| | (Acrylic acid/stearyl acrylate) copolymer *7 | | | | | | | | | | | | | |
| (B') | Acrylates copolymer *8 | | | | | | | 0.246 | 0.154 | 0.334 | | | | |
| | (Methyl vinyl ether/maleic acid) copolymer *9 | | | | | | | | | | 0.074 | 0.046 | 0.1 | 0.1 |
| (C) | Amodimethicone *10 | | | 0.2 | 0.2 | 0.2 | 0.01 | 0.2 | 0.2 | 0.2 | 0.2 | 0.2 | 0.2 | 0.2 |
| | (Bisisobutyl PEG-15/Amodimethicone) copolymer * 11 | | | | | | | | | | | | | |
| (D) | Stearoxypropyldimethylamine *12 | | 2.2 | 2.2 | 2.2 | 2.2 | 2.2 | 2.2 | 2.2 | 2.2 | 2.2 | 2.2 | 2.2 | 2.2 |
| (E) | Stearyl alcohol * 13 | | 6.38 | 6.38 | 6.38 | 6.38 | 6.38 | 6.38 | 6.38 | 6.38 | 6.38 | 6.38 | 6.38 | 6.38 |
| (F) | Dipropylene glycol *14 | | 2 | 2 | 2 | 2 | 2 | 2 | 2 | 2 | 2 | 2 | 2 | 2 |
| (G) | Dimethicone *15 | | 2 | 2 | 2 | 2 | 2 | 2 | 2 | 2 | 2 | 2 | 2 | 2 |
| | Lanolin fatty acid * 16 | | 1 | 1 | 1 | 1 | 1 | 1 | 1 | 1 | 1 | 1 | 1 | 1 |
| | Lactic acid *17 | | 1.3 | 1.3 | 1.3 | 1.3 | 1.3 | 1.3 | 1.3 | 1.3 | 1.3 | 1.3 | 1.3 | 1.3 |
| | Purified water | | Remaining amount | Remaining amount | Remaining amount | Remaining amount | Remaining amount | Remaining amount | Remaining amount | Remaining amount | Remaining amount | Remaining amount | Remaining amount | Remaining amount |
| Total | | | 100.00 | 100.00 | 100.00 | 100.00 | 100.00 | 100.00 | 100.00 | 100.00 | 100.00 | 100.00 | 100.00 | 100.00 |
| Component (A) effective content (% by mass) | | | 0.126 | 0.000 | 0.126 | 0.631 | 0.126 | 0.126 | 0.154 | 0.075 | 0.126 | 0.154 | 0.075 | 0.900 |
| Component (B) or (B') effective content (% by mass) | | | 0.074 | 0.074 | 0 | 0.369 | 0.074 | 0.0738 | 0.0462 | 0.1002 | 0.074 | 0.046 | 0.1 | 0.1 |
| Component (C) effective content (% by mass) | | | 0 | 0.20 | 0.20 | 0.20 | 0.01 | 0.20 | 0.20 | 0.20 | 0.20 | 0.20 | 0.20 | 0.20 |
| Component (D) effective content (% by mass) | | | 1.98 | 1.98 | 1.98 | 1.98 | 1.98 | 1.98 | 1.98 | 1.98 | 1.98 | 1.98 | 1.98 | 1.98 |
| Component (E) effective content (% by mass) | | | 6.60 | 6.60 | 6.60 | 6.60 | 6.60 | 6.60 | 6.60 | 6.60 | 6.60 | 6.60 | 6.60 | 6.60 |
| Component (G) effective content (% by mass) | | | 2.04 | 2.04 | 2.04 | 2.04 | 2.04 | 2.04 | 2.04 | 2.04 | 2.04 | 2.04 | 2.04 | 2.04 |
| Components (A)+((B) or (B')) effective content (% by mass) | | | 0.20 | 0.074 | 0.13 | 1.00 | 0.20 | 0.20 | 0.20 | 0.17 | 0.20 | 0.20 | 0.17 | 1.00 |
| Mass ratio [(A+B)/C] or [(A+B')/C] | | | - | 0.37 | 0.63 | 5.00 | 20.02 | 1.00 | 1.00 | 0.87 | 1.00 | 1.00 | 0.87 | 5.00 |
| Evaluation results | | Emulsified state | A | C | A | D | A | C | C | C | C | C | C | C |
| | | Storage stability | A | A | A | D | A | D | D | A | A | D | D | D |
| | | Feel at the time of application | 2.2 | -** | 1.8 | | 2.6 | -** | -** | -** | -** | -** | -** | -** |
| | | Moisture resistance of hair after treatment (tress width change value after storage under high humidity condition) | 1.3 | -** | 1.6 | -* | 1.6 | -** | -** | -** | -** | -** | -** | -** |

| | | | | | | | | | | | | | | |
|---|---|---|---|---|---|---|---|---|---|---|---|---|---|---|
| * Not evaluated due to separation at stability check ** Comparative Examples in which the emulsified state was evaluated as C were not evaluated. | | | | | | | | | | | | | | |

The components described in the Tables are as follows.

### <Component (A): Cationic Polymer or Amphoteric Polymer>

*1 Polyquaternium-6 (dimethyldiallylammonium polymer), "MERQUAT 100" manufactured by Lubrizol Advanced Materials, Inc., cationic charge density: 6.18 meq/g, Mw: 150,000, active ingredient amount: 41.5% by mass
*2 Polyquaternium-39 (acrylamide-acrylic acid-dimethyldiallylammonium chloride copolymer liquid), "MERQUAT 3940 POLYMER" manufactured by Lubrizol Advanced Materials, Inc., cationic charge density: 2.97 meq/g, Mw: 150,000, active ingredient amount: 43% by mass
*3 Polyquaternium-16 (vinylimidazolium trichloride-vinylpyrrolidone copolymer) "Luviquat FC 550" manufactured by BASF SE, cationic charge density: 3.91 meq/g, Mw: 80,000, active ingredient amount: 43% by mass
*4 Polyquaternium-22 (dimethyldiallylammonium chloride-acrylic acid copolymer liquid), "MERQUAT 280" manufactured by Lubrizol Advanced Materials, Inc., cationic charge density: 4.99 meq/g, Mw: 450,000, active ingredient amount: 41% by mass
*5 Polyquaternium-47 (acrylic acid-methyl acrylate-methacrylamidopropyltrimethyl ammonium chloride copolymer), "MERQUAT 2001 POLYMER" manufactured by Lubrizol Advanced Materials, Inc., cationic charge density: 3.21 meq/g, Mw: 1,300,000, active ingredient amount: 21% by mass

### <Component (B): Anionic Polymer>

*6 Sodium polyacrylate, "ACUSOL 445G Polymer" manufactured by The Dow Chemical Company, anionic charge density: 10.6 meq/g, Mw: 25,000, active ingredient amount: 100% by mass
*7 (Acrylic acid/stearyl acrylate) copolymer "SOFCARE SA-37W" manufactured by Kao Corporation, anionic charge density: 9.3 meq/g, Mw: 20,000, active ingredient amount: 10% by mass

### <Component (B'): Anionic Polymer Other than Component (B)>

*8 Acrylates copolymer, "Carbopol Aqua SF-1 Polymer" manufactured by Lubrizol Advanced Materials, Inc., Mw: > 500,000, active ingredient amount: 30% by mass
*9 (Methyl vinyl ether/maleic acid) copolymer, "Gantrez S-97" manufactured by Ashland Specialty Ingredients, active ingredient amount: 100% by mass

### <Component (C): Amino-Modified Silicone/Aminopolyether-Modified Silicone>

*10 Amodimethicone, "Silicone SF 8457 C" manufactured by Dow Toray Co., Ltd., active ingredient amount: 100% by mass
*11 (Bisisobutyl PEG-15/Amodimethicone) copolymer, "DOWSIL SS-3588 FLUID" manufactured by Dow Toray Co., Ltd., Si/EO (molar ratio): 2.39, active ingredient amount: 71.5% by mass

### <Component (D): Cationic Surfactant>

*12 Stearoxypropyldimethylamine, "FARMIN DM-E80" manufactured by Kao Corporation, active ingredient amount: 90% by mass, mixture of stearyl alcohol 10% by mass <Component (E): Higher Alcohol>
* 13 Stearyl alcohol, "KALCOL 8098" manufactured by Kao Corporation, active ingredient amount: 100% by mass

### <Component (F): Non-Aromatic Polyol>

*14 Dipropylene glycol, "DPG-RF" manufactured by ADEKA CORPORATION, active ingredient amount: 100% by mass

### <Component (G): Oil Agent>

*15 Dimethicone, "Silicone KHS-3" manufactured by Shin-Etsu Chemical Co., Ltd., active ingredient amount: 100% by mass
*16 Lanolin fatty acid, "18MEA-CL1" manufactured by Kao Corporation, active ingredient amount: 3.5% by mass

### <Organic Acid>

*17 Lactic acid, "PURAC ULTRAPURE 90" manufactured by PURAC Tiland Ltd., active ingredient amount: 90% by mass

### Industrial Applicability

According to the present invention, a hair cosmetic composition which has a good feel at the time of application to the hair, can suppress the spread of the hair under high humidity conditions, and has high stability can be provided.

## Claims

1. A hair cosmetic composition comprising:
component (A): one or more polymers selected from the group consisting of a cationic polymer and an amphoteric polymer;
component (B): one or more anionic polymers selected from the group consisting of a polyacrylic acid, a (meth)acrylic acid/alkyl (meth)acrylate copolymer, and a salt thereof; and
component (C): one or more modified silicones selected from the group consisting of an amino-modified silicone and an aminopolyether-modified silicone,
wherein the component (B) has a weight average molecular weight of 3,000 or more and 50,000 or less, a total content of the component (A) and the component (B) is less than 1.0% by mass, and a content of the component (C) is 0.03% by mass or more.

2. The hair cosmetic composition according to claim 1, wherein the total content of the component (A) and the component (B) in the hair cosmetic composition is 0.05% by mass or more and 0.9% by mass or less.

3. The hair cosmetic composition according to claim 1 or 2, wherein the content of the component (C) in the hair cosmetic composition is 0.05% by mass or more and 5.0% by mass or less.

4. The hair cosmetic composition according to any one of claims 1 to 3, further comprising a cationic surfactant as component (D).

5. The hair cosmetic composition according to any one of claims 1 to 4, further comprising a higher alcohol as component (E).

6. The hair cosmetic composition according to any one of claims 1 to 5, wherein the hair cosmetic composition is an emulsion composition.
